# EUROPEAN PATENT SPECIFICATION

(11) **EP 1 578 409 B1**
(45) Date of publication and mention of the grant of the patent: **08.12.2010**
(21) Application number: 03818838.9
(22) Date of filing: 17.12.2003
(51) Int. Cl.: A61K 31/519, A61K 31/00, A61K 45/06, A61P 25/14

(54) **USE OF ADENOSINE A2A RECEPTOR ANTAGONISTS FOR TREATMENT OR PREVENTION OF EXTRA-PYRAMIDAL SYNDROME**
VERWENDUNG VON A2A REZEPTOR-ANTAGONISTEN ZUR BEHANDLUNG DES EXTRAPYRAMIDALEN SYNDROMS
UTILISATION D'ANTAGONISTES DES RECEPTEURS DE L'ADENOSINE A2A POUR LE TRAITEMENT DU SYNDROME EXTRAPYRAMIDAL

(30) Priority: 19.12.2002 US 435321 P
(43) Date of publication of application: 28.09.2005
(60) Divisional application: 10186087.2
(73) Proprietor: Schering Corporation, Kenilworth, NJ 07033-0530 (US)
(72) Inventor: GRZELAK, Michael, Wayne, NJ 07470 (US); HUNTER, John, C., Warren, NJ 07059 (US); POND, Annamarie, Budd Lake, NJ 07828 (US); VARTY, Geoffrey, Cranford, NJ 07016 (US)
(74) Representative: Hussain, Deeba
(86) International application number: PCT/US2003/040456
(87) International publication number: WO 2005/044245

(56) References cited:
- WO-A-00/13682
- WO-A-01/02409
- WO-A-01/92264
- WO-A-01/92264
- WO-A-99/26627
- WO-A-02/055083
- WO-A-02/080957
- WO-A-03/022283
- WO-A-03/048163
- WO-A-03/048163
- WO-A-03/048165
- WO-A-03/063876
- WO-A-03/063876
- WO-A-2004/019949
- WO-A-2004/094431
- RICHTER ANGELIKA ET AL: "Effects of adenosine receptor agonists and antagonists in a genetic animal model of primary paroxysmal dystonia" BRITISH JOURNAL OF PHARMACOLOGY, vol. 134, no. 2, September 2001 (2001-09), pages 343-352, XP002400245 ISSN: 0007-1188
- DEL CARMEN PARRA CID M ET AL: "A NEW DERIVATE OF THE XANTHINE: A15BU IMPROVE THE MOTOR FUNCTION." SOCIETY FOR NEUROSCIENCE ABSTRACT VIEWER AND ITINERARY PLANNER, vol. 2002, November 2002 (2002-11), pages Abstract No. 165.15 URL-http://sf, XP008068918 & 32ND ANNUAL MEETING OF THE SOCIETY FOR NEUROSCIENCE; ORLANDO, FLORIDA, USA; NOVEMBER 02-07, 2002
- DE SARRO GIOVAMBATTISTA ET AL: "Repeated treatment with adenosine A-1 receptor agonist and antagonist modifies the anticonvulsant properties of CPPene" EUROPEAN JOURNAL OF PHARMACOLOGY, vol. 317, no. 2-3, 1996, pages 239-245, XP002400246 ISSN: 0014-2999
- KHAN GHOUS M ET AL: "Anticonvulsant effect and neurotransmitter modulation of focal and systemic 2-chloroadenosine against the development of pilocarpine-induced seizures" NEUROPHARMACOLOGY, vol. 39, no. 12, 13 September 2000 (2000-09-13), pages 2418-2432, XP002400247 ISSN: 0028-3908
- SEUBERT CHRISTOPH N ET AL: "Midazolam selectively potentiates the A2A- but not A1-receptor-mediated effects of adenosine: Role of nucleoside transport inhibition and clinical implications" ANESTHESIOLOGY (HAGERSTOWN), vol. 92, no. 2, February 2000 (2000-02), pages 567-577, XP008068919 ISSN: 0003-3022
- CHARTOFF ELENA H ET AL: "Role of adenosine and N-methyl-D-aspartate receptors in mediating haloperidol-induced gene expression and catalepsy" JOURNAL OF PHARMACOLOGY AND EXPERIMENTAL THERAPEUTICS, vol. 291, no. 2, November 1999 (1999-11), pages 531-537, XP002302029 ISSN: 0022-3565
- WARD R P ET AL: "MOLECULAR AND BEHAVIORAL EFFECTS MEDIATED BY GS-COUPLED ADENOSINE A2A, BUT NOT SEROTONIN 5-HT4 OR 5-HT6 RECEPTORS FOLLOWING ANTIPSYCHOTIC ADMINISTRATION" NEUROSCIENCE, NEW YORK, NY, US, vol. 89, no. 3, March 1999 (1999-03), pages 927-938, XP008035954 ISSN: 0306-4522
- PINNA A ET AL: "INVOLVEMENT OF ADENOSINE A2A RECEPTORS IN THE INDUCTION OF C-FOS EXPRESSION BY CLOZAPINE AND HALOPERIDOL" NEUROPSYCHOPHARMACOLOGY, ELSEVIER SCIENCE PUBLISHING, NEW YORK, NY, US, vol. 20, no. 1, January 1991 (1991-01), pages 44-51, XP008035960 ISSN: 0893-133X
- WARDAS J ET AL: "SCH 58261, AN A2A ADENOSINE RECEPTOR ANTAGONIST, COUNTERACTS PARKINSONIAN-LIKE MUSCLE RIGIDITY IN RATS" SYNAPSE, WILEY AND SONS, CHICHESTER, GB, vol. 41, no. 2, 14 May 2001 (2001-05-14), pages 160-171, XP008035144 ISSN: 0887-4476
- MORELLI M ET AL: "ADENOSINE A2A RECEPTOR ANTAGONISTS: POTENTIAL THERAPEUTIC AND NEUROPROTECTIVE EFFECTS IN PARKINSON'S DISEASE" NEUROTOXICITY RESEARCH, HARWOOD ACADEMIC PUBLISHERS, LAUSANNE, CH, vol. 3, no. 6, 2001, pages 545-556, XP008035146 ISSN: 1029-8428
- KANDA T ET AL: "KF17837: a novel selective adenosine A2A receptor antagonist with anticataleptic activity." EUROPEAN JOURNAL OF PHARMACOLOGY. 2 MAY 1994, vol. 256, no. 3, 2 May 1994 (1994-05-02), pages 263-268, XP002302030 ISSN: 0014-2999
- KUWAMA Y ET AL: "A2A ADENOSINE RECEPTOR ANTAGONISTS ARE ANTIPARKINSONIAN IN ANIMAL MODELS" ABSTRACTS OF THE SOCIETY FOR NEUROSCIENCE, SOCIETY FOR NEUROSCIENCE, WASHINGTON, DC, US, vol. 23, no. 1/2, 1997, page 297, XP008036055 ISSN: 0190-5295
- SHIOZAKI S ET AL: "ACTIONS OF ADENOSINE A2A RECEPTOR ANTAGONIST KW-6002 ON DRUG-INDUCED CATALEPSY AND HYPOKINESIA CAUSED BY RESERPINE OR MPTP" PSYCHOPHARMACOLOGY, SPRINGER VERLAG, BERLIN, DE, vol. 147, no. 1, 1999, pages 90-95, XP001077769 ISSN: 0033-3158
- ADAMI M ET AL: "THE ADENOSINE A2A RECEPTOR BLOCKER, SCH 58261, IS EFFECTIVE OVER A 2-WEEK TREATMENT IN HALOPERIDOL-INDUCED CATALEPSY" BRITISH JOURNAL OF PHARMACOLOGY, BASINGSTOKE, HANTS, GB, vol. 126, no. PROC SUPPL, March 1999 (1999-03), page ABSTRNO283P, XP008035971 ISSN: 0007-1188
- ONGINI E ET AL: "DUAL ACTIONS OF A2A ADENOSINE RECEPTOR ANTAGONISTS ON MOTOR DYSFUNCTION AND NEURODEGENERATIVE PROCESSES" DRUG DEVELOPMENT RESEARCH, NEW YORK, NY, US, vol. 52, no. 1/2, 2001, pages 379-386, XP008035967 ISSN: 0272-4391
- ONGINI E ET AL: "NEUROPHARMACOLOGY OF THE ADENOSINE A2A RECEPTORS" DRUG DEVELOPMENT RESEARCH, NEW YORK, NY, US, vol. 39, no. 3/4, 1996, pages 450-460, XP008035966 ISSN: 0272-4391
- CORREA M ET AL: "THE ADENOSINE A2A ANTAGONIST KF17837 REVERSES THE LOCOMOTOR SUPPRESSION AND TREMULOUS JAW MOVEMENTS INDUCED BY HALOPERIDOL IN RATS: POSSIBLE RELEVANCE TO PARKINSONISM." SOCIETY FOR NEUROSCIENCE ABSTRACT VIEWER AND ITINERARY PLANNER, vol. 2002, 2002, page ABSTRACT NO. 885.8, XP008035957 & 32ND ANNUAL MEETING OF THE SOCIETY FOR NEUROSCIENCE; ORLANDO, FLORIDA, USA; NOVEMBER 02-07, 2002
- PARSONS B ET AL: "NEUROLEPTICS UP-REGULATE ADENOSINE A2A RECEPTORS IN RAT STRIATUM: IMPLICATIONS FOR THE MECHANISM AND THE TREATMENT OF TARDIVE DYSKINESIA" JOURNAL OF NEUROCHEMISTRY, NEW YORK, NY, US, vol. 65, no. 5, November 1995 (1995-11), pages 2057-2064, XP008035958 ISSN: 0022-3042
- MALLY J ET AL: "POTENTIAL ROLE OF ADENOSINE ANTAGONIST THERAPY IN PATHOLOGICAL TREMOR DISORDERS" PHARMACOLOGY AND THERAPEUTICS, ELSEVIER, GB, vol. 72, no. 3, 1996, pages 243-250, XP002930163 ISSN: 0163-7258
- FERRE S ET AL: "GLUTAMATE MGLU5-ADENOSINE A2A-DOPAMINE D2 RECEPTOR INTERACTIONS IN THE STRIATUM. IMPLICATIONS FOR DRUG THERAPY IN NEURO-PSYCHIATRIC DISORDERS AND DRUG ABUSE" CURRENT MEDICINAL CHEMISTRY, BENTHAM SCIENCE PUBLISHERS BV, BE, vol. 3, no. 1, 2003, pages 1-26, XP008035176 ISSN: 0929-8673
- CASEY DANIEL E: "Serotonergic and dopaminergic aspects of neuroleptic-induced extrapyramidal syndromes in nonhuman primates" PSYCHOPHARMACOLOGY, vol. 112, no. 1 SUPPL., 1993, pages S55-S59, ISSN: 0033-3158
- VARTY GEOFFREY B ET AL: "The effects of adenosine A2A receptor antagonists on haloperidol-induced movement disorders in primates." PSYCHOPHARMACOLOGY OCT 2008 LNKD- PUBMED:18594798, vol. 200, no. 3, October 2008 (2008-10), pages 393-401, ISSN: 0033-3158

## Description

### FIELD OF THE INVENTION

The present invention relates to the use of certain adenosine A₂ₐ receptor antagonists for the manufacture of a medicament for the treatment of a variety of neurological syndromes involving the extra-pyramidal motor system (*i.e.* Extra-Pyramidal Syndrome) that occur following the acute and chronic use of almost all antipsychotic drugs as defined in the claims.

### BACKGROUND OF THE INVENTION

Extra-Pyramidal Syndrome (EPS) is a collective term for a series of adverse neurological reactions associated with the use of antipsychotic drugs. There are six different categories of EPS-related neurological syndromes of which four, dystonia, akathisia, pseudoparkinsonism (parkinsonian syndrome), and tardive dyskinesia, are particularly prevalent in patients taking antipsychotic medication. Dystonia is a painful spasm of the muscle groups of, in particular, the neck, jaw, back, pharynx, and larynx. It is most common in young males being treated with antipsychotic drugs, but can also be associated with the use of cocaine, tricyclic antidepressants, lithium and anticonvulsants such as phenytoin and carbamazepine. Pseudoparkinsonism manifests itself as akinesia (rigidity, stiffness and slow voluntary motion, stooped, shuffling walk) and tremor and these symptoms develop within weeks or months after initiation of therapy. Akathisia manifests itself as strong, subjective inner feelings of distress or discomfort characterized by motor restlessness. Often mistaken for agitation or anxiety, this common syndrome is frequently under-diagnosed and is the least responsive to treatment. Tardive dyskinesia is a late-appearing syndrome associated with chronic use of neuroleptic drugs. It occurs more frequently in older patients and is characterized by stereotypical, repetitive, involuntary, quick choreiform movements of the face, eyelids, mouth, tongue, extremities and trunk.

EPS is more prevalent with the use of typical antipsychotic agents but has also been reported with the use of atypical agents. Typical antipsychotics include loxapine, haloperidol, chlorpromazine, prochlorperazine and thiothixene. Atypical antipsychotics include clozapine, olanzapine, loxapine, quetiapine, ziprasidone and risperidone.

Akathisia is also a characteristic of RLS and PLMS, as well as PLMD (periodic leg (or limb) movement disorder). RLS is a common disorder that causes patients to have an irresistible and unpleasant desire to move their legs; it usually manifests during periods of inactivity and/or at night, and can disturb sleep. Patients who do not have the typical RLS symptoms, but who do exhibit periodic leg movements that adversely impact sleep, are diagnosed with PLMS. Treatments for RLS and PLMS have included levodopa/carbidopa, levodopa/benserazide, dopamine agonists such as pramipexole and ropinerole, benzodiazepines, opioids, anticonvulsants and iron (ferrous sulfate). RLS and PLMS have been extensively described in the literature, for example by Saletu et al, Neuropsychobiology, 41, 4 (2000), p. 190-9.

The purine nucleotide, adenosine, is known to be an endogenous modulator of a number of physiological functions in the central (CNS) and peripheral nervous systems.

Adenosine exerts its biological actions through a class of membrane specific receptors which belong to the super family of receptors coupled with G proteins. Biochemical and pharmacological studies, together with advances in molecular biology, have allowed the identification of at least four subtypes of adenosine receptors: A₁, A₂ₐ, A_{2b} and A₃. Analogs of adenosine able to interact as antagonists with the A₁, A₂ₐ, A_{2b} and A₃ receptors have also been identified.

In the CNS, data has shown that A₂ₐ receptors are present in high density in the basal ganglia, known to be important in the control of fine motor movement. Moreover, selective antagonists for the A₂ₐ receptor are of pharmacological interest because of their demonstrated efficacy in reducing motor impairment thereby improving function in neurodegenerative diseases such as Parkinson's disease and related movement disorders (*e.g.* Huntington's Disease). A₂ₐ antagonists appear to demonstrate a reduced side-effect liability (*e.g.* no dyskinesia) compared to current dopaminergic therapies resulting in an improved therapeutic index. A₂ₐ antagonists may also have antidepressant properties and stimulate cognitive functions. Some xanthine-related compounds have been found to be A₁ receptor selective antagonists, and xanthine and non-xanthine compounds have been found to have high A₂ₐ affinity with varying degrees of A₂ₐ vs. A₁ selectivity. Adenosine A₂ₐ receptor antagonists have been disclosed previously, for example in WO 95/01356, US 6,630,476 and WO 01/92264.

Hoffman D.C., Donovan H. : "Catalepsy as a rodent model for detecting drugs with extrapyramidal side effect liability"; Psychopharmacology, vol. 120, no 2, pages 128-133 discloses catalepsy tests conducted on rats using antipsychotic drugs and concludes that catalepsy is a suitable rodent model for detecting compounds with EPS liability in humans.

### SUMMARY OF THE INVENTION

The present invention relates to the use of an A₂ₐ receptor antagonist for the preparation of a medicament for the treatment or prevention of Extra-Pyramidal Syndrome (e.g., dystonia, akathisia, pseudoparkinsonism and tardive dyskinesia) in patients treated with an antipsychotic agent that has the side effect of inducing EPS, wherein the adenosine A₂ₐ antagonist is a compound of formula I as defined in the claims. The adenosine A₂ₐ receptor antagonist can be administered after the symptoms of EPS have manifested, or an adenosine A₂ₐ receptor antagonist can be administered at the onset of administering an antipsychotic agent in order to prevent EPS from occurring. The disclosure, therefore, also includes treating or preventing EPS induced by an antipsychotic agent comprising administering a combination of an antipsychotic agent and an adenosine A₂ₐ antagonist to a patient in need thereof.

The disclosure also relates to the treatment of primary (idiopathic) dystonia, and to the treatment or prevention of dystonia in patients who exhibit dystonia as a result of treatment with a tricyclic antidepressant, lithium or an anticonvulsant, or who have used cocaine, comprising administering a therapeutically effective amount of an adenosine A₂ₐ receptor antagonist to a patient in need thereof. When dystonia is caused by treatment with a tricyclic antidepressant, lithium or an anticonvulsant, the adenosine A₂ₐ receptor antagonist can be administered after the symptoms of dystonia have manifested, or an adenosine A₂ₐ receptor antagonist can be administered at the onset of administering a tricyclic antidepressant, lithium or an anticonvulsant in order to prevent dystonia from occurring. The disclosure, therefore, also includes treating or preventing dystonia induced by a tricyclic antidepressant, lithium or an anticonvulsant comprising administering a combination of an adenosine A₂ₐ antagonist and a tricyclic antidepressant, lithium or an anticonvulsant to a patient in need thereof.

The disclosure also relates to the treatment of RLS or PLMS, comprising administering to a patient In need thereof a therapeutically effective amount of an adenosine A₂ₐ receptor antagonist. The disclosure also comprises treating RLS or PLMS comprising administering a combination of an adenosine A₂ₐ antagonist with another agent useful In treating RLS or PLMS, such as levodopa/carbidopa, levodopa/benserazide, a dopamine agonist, a benzodiazepine, an opioid, an anticonvulsant or iron, to a patient in need thereof.

In an embodiment, this invention relates to a kit comprising, in separate containers in a single package, pharmaceutical compositions for use in combination to treat or prevent EPS caused by treatment with antipsychotic agent, wherein one container comprises a pharmaceutical composition comprising an effective amount of an adenosine A₂ₐ receptor antagonist which is a compound of formula I as defined in claim 1, in a pharmaceutically acceptable carrier, and wherein a separate container comprises a pharmaceutical composition comprising an effective amount of an antipsychotic agent.

In another instance, this disclosure relates to a kit comprising, in separate containers in a single package, pharmaceutical compositions for use in combination to treat or prevent dystonia caused by treatment with a tricyclic antidepressant, lithium or an anticonvulsant, wherein one container comprises a pharmaceutical composition comprising an effective amount of an adenosine A₂ₐ receptor antagonist in a pharmaceutically acceptable carrier, and wherein a separate container comprises a pharmaceutical composition comprising an effective amount of a tricyclic antidepressant, lithium or an anticonvulsant.

In another instance, this disclosure relates to a kit comprising, in separate containers in a single package, pharmaceutical compositions for use in combination to treat RLS or PLMS, wherein one container comprises a pharmaceutical composition comprising an effective amount of an adenosine A₂ₐ receptor antagonist in a pharmaceutically acceptable carrier, and wherein a separate container comprises a pharmaceutical composition comprising an effective amount of levodopa/carbidopa, ievodopa/benserazide, a dopamine agonist, a benzodiazepine, an opioid, an anticonvulsant or iron.

The disclosure also relates to the use of an adenosine A₂ₐ receptor antagonist for the preparation of a medicament for treating or preventing EPS, dystonia, RLS or PLMS, alone or in combination with the other agents discussed above.

### DETAILED DESCRIPTION OF THE DRAWINGS

A more complete understanding of the invention may be obtained by reading the following description in conjunction with the appended figures relating to haloperidol-induced EPS in *Cebus apella* monkeys.

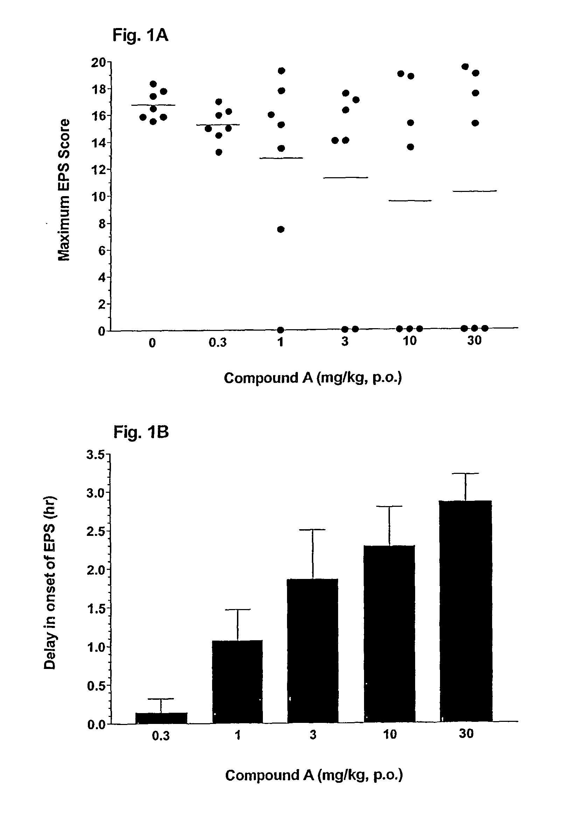

Figure 1A illustrates the effect of Compound A (1-30 mg/kg, p.o.) on maximum EPS score.
Figure 1B represents the mean delay in onset of EPS for each treatment group compared to a vehicle control group.

### DETAILED DESCRIPTION OF THE INVENTION

The adenosine A₂ₐ receptor antagonist useful in the present invention has structural formula I, as defined below and in claim 1. Adenosine A₂ₐ receptor antagonists can be identified by the binding assay described below. Specific examples of adenosine A₂ₐ antagonists include the compounds disclosed in several patents and patent applications, e.g. WO 95/01356; US 5,565,460; US 6,630,475 B2; US 5,935,964; WO 03/032996; WO 03/048165; WO 03/048164; WO 03/048163; and WO 01/02409. Specifically, these patents and applications disclose the following compounds.

US 6,630,475 B2 discloses compounds having the structural formula I 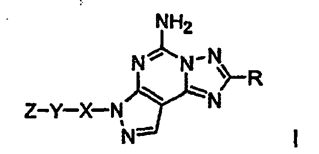 or a pharmaceutically acceptable salt thereof, wherein
R is R¹-furanyl, R¹-thienyl, R¹-pyrldyl, R¹-pyridyl N-oxide, R¹-oxazolyl, R¹⁰-phenyl, R¹-pyrrolyl or C₄-C₆ cycloalkenyl;
X is C₂-C₆ alkylene or -C(O)CH₂-;
Y is -N(R²)CH₂CH₂N(R³)-, -OCH₂CH₂N(R²)-, -O-, -S-, -CH₂S-, -(CH₂)₂-NH-, or 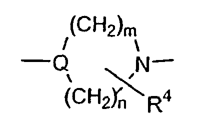 and
Z is R⁵-phenyl, R⁵-phenyl(C₁-C₆)alkyl, R⁵-heteroaryl, diphenylmethyl, R⁶-C(O)-, R⁶-SO₂-, R⁶-OC(O)-, R⁷-N(R⁸)-C(O)-, R⁷-N(R⁸)-C(S)-, 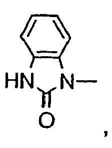 phenyl-CH(OH)-, or phenyl-C(=NOR²)-; or when Q is 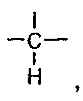 Z is also phenylamino or pyridylamino; or
Z and Y together are 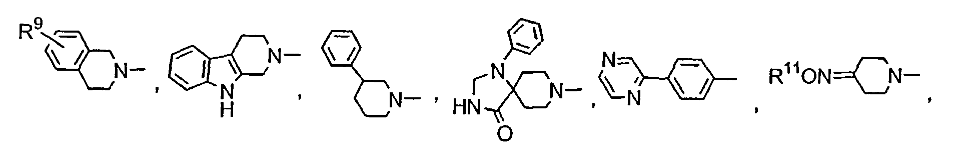 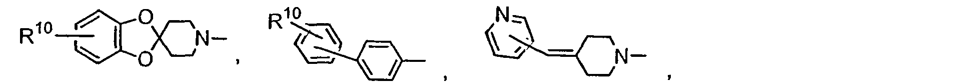 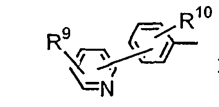 or an N-oxide thereof, 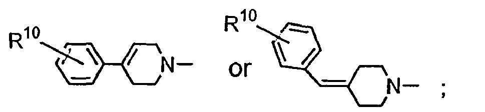 R¹ is 1 to 3 substituents independently selected from hydrogen, C₁-C₆-alkyl, -CF₃, halogen, -NO₂, -NR¹²R¹³, C₁-C₆ alkoxy, C₁-C₆ alkylthio, C₁-C₆ alkylsulfinyl, and C₁-C₆ alkylsulfonyl;
R² and R³ are independently selected from the group consisting of hydrogen and C₁-C₆ alkyl;
m and n are independently 2-3;
Q is 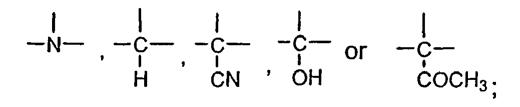 R⁴ is 1-2 substituents independently selected from the group consisting of hydrogen and C₁-C₆alkyl, or two R⁴ substituents on the same carbon can form =O;
R⁵ is 1 to 5 substituents independently selected from the group consisting of hydrogen, halogen, C₁-C₆ alkyl, hydroxy, C₁-C₆ alkoxy, -CN, di-((C₁-C₆)alkyl)amino, -CF₃, -OCF₃, acetyl, -NO₂, hydroxy(C₁-C₆)alkoxy, (C₁-C₆)-alkoxy(C₁-C₆)alkoxy, di-((C₁-C₆)-alkoxy)(C₁-C₆)alkoxy, (C₁-C₆)-alkoxy(C₁-C₆)alkoxy-(C₁-C₆)-alkoxy, carboxy(C₁-C₆)-alkoxy, (C₁-C₆)-alkoxycarbonyl(C₁-C₆)alkoxy, (C₃-C₆)cycloalkyl(C₁-C₆)alkoxy, di-((C₁-C₆)alkyl)amino(C₁-C₆)alkoxy, morpholinyl, (C₁-C₆)alkyl-SO₂-, (C₁-C₆)alkyl-SO-(C₁-C₆)alkoxy, tetrahydropyranyloxy, (C₁-C₆)alkylcarbonyl(C₁-C₆)-alkoxy, (C₁-C₆)-alkoxycarbonyl, (C₁-C₆)alkylcarbonyloxy(C₁-C₆)-alkoxy, -SO₂NH₂, phenoxy, 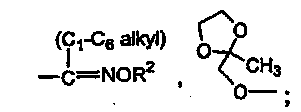 or adjacent R⁵ substituents together are -O-CH₂-O-, -O-CH₂CH₂-O-, -O-CF₂-O- or -O-CF₂CF₂-O- and form a ring with the carbon atoms to which they are attached;
R⁶ is (C₁-C₆)alkyl, R⁵-phenyl, R⁵-phenyl(C₁-C₆)alkyl, thienyl, pyridyl, (C₃-C₆)-cycloalkyl, (C₁-C₆)alkyl-OC(O)-NH-(C₁-C₆)alkyl-, di-((C₁-C₆)alkyl)aminomethyl, or 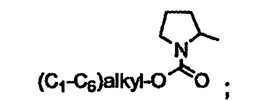 R⁷ is (C₁-C₆)alkyl, R⁵-phenyl or R⁵-phenyl(C₁-C₆)alkyl;
R⁸ is hydrogen or C₁-C₆ alkyl; or R⁷ and R⁸ together are -(CH₂)ₚ-A-(CH₂)_{q}, wherein p and q are independently 2 or 3 and A is a bond, -CH₂-, -S- or -O-, and form a ring with the nitrogen to which they are attached;
R⁹ is 1-2 groups independently selected from hydrogen, C₁-C₆ alkyl, hydroxy, C₁-C₆ alkoxy, halogen, -CF₃ and (C₁-C₆)alkoxy(C₁-C₆)alkoxy ;
R¹⁰ is 1 to 5 substituents Independently selected from the group consisting of hydrogen, halogen, C₁-C₆ alkyl, hydroxy, C₁-C₆ alkoxy, -CN, -NH₂, C₁-C₆alkylamlno, di-((C₁-C₆)alkyl)amino, -CF₃, -OCF₃ and -S(O)₀₋₂(C₁-C₆)alkyl;
R¹¹ is H, C₁-C₆ alkyl, phenyl, benzyl, C₂-C₆ alkenyl, C₁-C₆ alkoxy(C₁-C₆)alkyl, di-((C₁-C₆)alkyl)amino(C₁-C₆)alkyl, pyrrolidinyl(C₁-C₆)alkyl or piperidino(C₁-C₆)alkyl;
R¹² is H or C₁-C₆ alkyl; and
R¹³ is (C₁-C₆)alkyl-C(O)- or (C₁-C₆)alkyl-SO₂-.

Preferred compounds of formula I are those wherein R is R¹-furanyl, R¹-thienyl, R¹-pyrrolyl or R¹⁰-phenyl, more preferably R¹-furanyl. R¹ is preferably hydrogen or halogen. Another group of preferred compounds is that wherein X is alkylene, preferably ethylene. Y is preferably 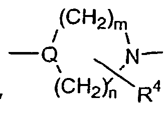 wherein Q is 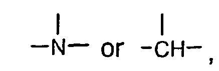 with Q preferably being nitrogen. Preferably, m and n are each 2, and R⁴ is H. A preferred definition for Z is R⁵-phenyl, R⁵-heteroaryl, R⁶-C(O)- or R⁶-SO₂-. R⁵ is preferably H, halogen, alkyl, alkoxy, hydroxyalkoxy or alkoxyalkoxy. R⁶ is preferably R⁵-phenyl.

Preferred specific compounds of formula I are those of the formula IA 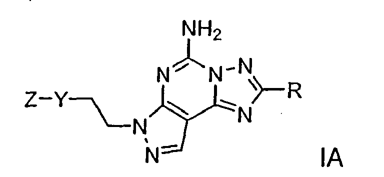 wherein R and Z-Y are as defined in the following table:

| Z-Y- | R |
|---|---|
| 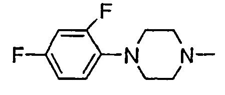 | 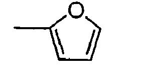 |
| 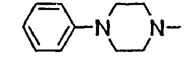 | 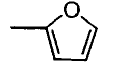 |
| 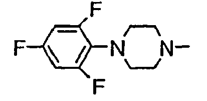 | 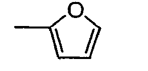 |
| 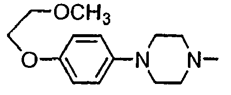 | 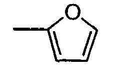 |
| 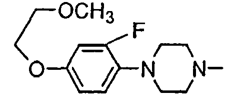 | 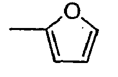 |
| 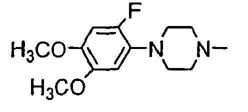 | 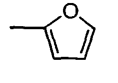 |
| 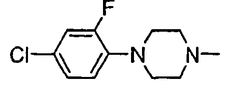 | 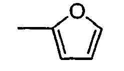 |
| 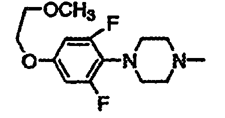 | 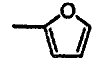 |
| 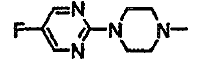 | 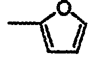 |
| 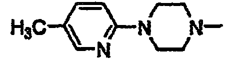 | 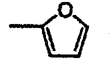 |
| 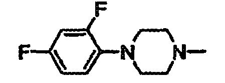 | 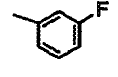 |
| 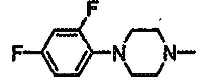 | 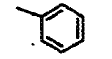 |

The present invention is defined in the appended claims.

The present invention relates to the use of an adenosine A₂ₐ receptor of formula I for the preparation of a medicament for the treatment or prevention of EPS in a patient treated with an antipsychotic agent that has the side effect of inducing EPS.

Other adenosine A₂ₐ receptor antagonists include those disclosed in WO 95/01356 as compounds having the structural formula II 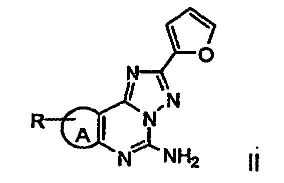 wherein:
A is pyrazole, imidazole or a triazole ring;
R is hydrogen; C₁-C₈ alkyl; C₃-C₇ alkenyl; C₃-C₇ alkynyl; C₃-C₇ cycloalkyl; C₁-C₅ alkyl substituted with one or more halogen atoms, hydroxy groups, C₁-C₄ alkoxy, C₃-C₇ cycloalkyl, groups of formula -NR₁R₂, -CONR₁R₂; aryl optionally substituted with halogen atoms, C₁-C₄ alkoxy groups, C₁-C₄ alkyl, nitro, amino, cyano, C₁-C₄ haloalkyl, C₁-C₄ haloalkoxy, carboxy, carboxyamido; C₇-C₁₀ aralkyl in which the aryl moiety can be substituted with one or more of the substituents indicated above for the aryl group; a group of formula -(CH₂)ₘ-Het, wherein Het is a 5-6 membered aromatic or non aromatic heterocyclic ring containing one or more heteroatoms selected from N, O, S and m is an integer from 1 to 5;
R₁, R₂ which are the same or different, are hydrogen, C₁-C₅ alkyl, C₇-C₁₀ aralkyl, phenyl, or taken together with the nitrogen they are linked to, form an azetidine ring or a 5-6 membered heterocyclic ring containing one or more heteroatoms such as N, O, S and n is an integer from 2 to 5.

Exemplary compounds of formula II are those wherein R is hydrogen, C₁-C₈ alkyl, aryl or C₇-C₁₀ aralkyl optionally substituted, preferably with halogen atoms.

US 5,935,964 discloses adenosine A₂ₐ receptor antagonist compounds having the structural formula III 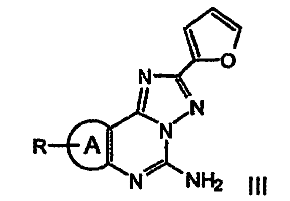 wherein A is pyrazole, imidazole or triazole ring;
R is 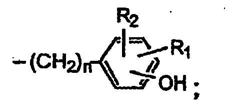 R₁ and R₂, which are the same or different, are H, OH, halogen, C₁-C₄ alkoxy, C₁-C₄ alkyl, nitro, amino, cyano, C₁-C₄ haloalkyl, C₁-C₄ haloalkoxy, carboxy or carboxamido; or the OH group, together with one of R₁ or R₂, or R₁ and R₂, can form a methylenedioxy group -O-CH₂-O-; and
n is an integer from 0-4.

Exemplary compounds of formula III are those wherein A is pyrazolo[4,3-e] or 1,2,3-triazolo[5,4-e].

US 5,565,460 discloses adenosine A₂ₐ receptor antagonist compounds having the structural formulas IVA and IVB, wherein formula IVA is 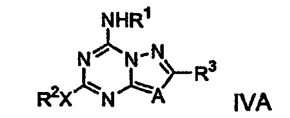 wherein R¹ represents hydrogen, substituted or unsubstituted lower alkyl, or substituted or unsubstituted lower alkanoyl;

R² represents hydrogen, substituted or unsubstituted lower alkyl, substituted or unsubstituted lower alkenyl, substituted or unubstituted cycloalkyl, substituted or unsubstituted aryl, substituted or unsubstituted aralkyl, or a substituted or unsubstituted heterocyclic group;
R³ represents a substituted or unsubstituted heterocyclic group;
X represents a single bond, O, S, S(O), S(O)₂, or NR⁴ ( in which R⁴ represents hydrogen, or substituted or unsubstituted lower alkyl; or R² and NR⁴ are combined to form a substituted or unsubstituted 4 to 6-membered saturated heterocyclic group): and
A represents N or CR⁵ (in which R⁵ represents hydrogen, or a substituted or unsubstituted lower alkyl); and
wherein formula IVB is 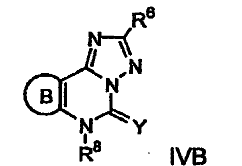 wherein R⁶ represents substituted or unsubstituted aryl, or a substituted or unsubstituted heterocyclic group;
Y represents O, S, or NR⁷ (in which R⁷ represents substituted or unsubstituted lower alkyl, substituted or unubstituted cycloalkyl, or substituted or unsubstituted aryl);
R⁸ represents hydrogen, substituted or unsubstituted lower alkyl, substituted or unsubstituted lower alkenyl, substituted or unsubstituted lower alkynyl, substituted or unubstituted cycloalkyl, substituted or unsubstituted aryl, substituted or unsubstituted aralkyl, or a substituted or unsubstituted heterocyclic group; and
B and the adjacent two carbon atoms are combined to form a substituted or unsubstituted, partially saturated or unsaturated, monocyclic or bicyclic, carbocyclic or heterocyclic group.
WO 03/032996 discloses adenosine A₂ₐ receptor antagonist compounds having the structural formula V 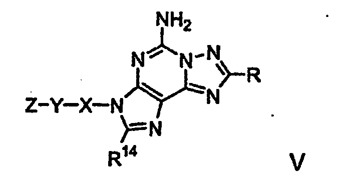 or a pharmaceutically acceptable salt thereof, wherein
R is R¹-heteroaryl, R¹⁰-phenyl, C₄-C₆ cycloalkenyl, -C(=CH₂)CH₃, -C≡C-CH₃, -C≡C-CH₂-OR², -CH=C(CH₃)₂, 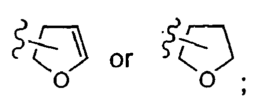 X is C₁-C₆ alkylene, -C(O)CH₂- or -C(O)N(R²)CH₂-;
Y is -N(R²)CH₂CH₂N(R³)-, -OCH₂CH₂N(R²)-, -O-, -S-, -CH₂S-, -(CH₂)₂₋₃-N(R²)-, R⁵-divalent heteroaryl, 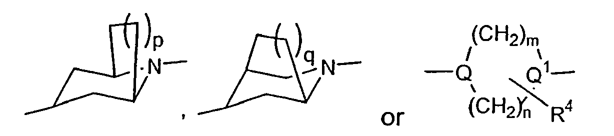 and
Z is R⁵-phenyl, R⁵-phenyl(C₁-C₆)alkyl, R⁵-heteroaryl, R⁵-bicyclic heteroaryl, R⁵-benzofused heteroaryl, diphenylmethyl or R⁶-C(O)-;
or when Y is 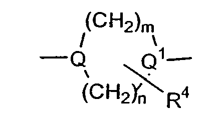 Z is also R⁶-SO₂-, R⁷-N(R⁸)-C(O)-, R⁷-N(R⁸)-C(S)- or R⁶OC(O)-;
or when Q is 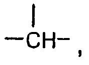 Z is also phenylamino or pyridylamino;
or Z and Y together are 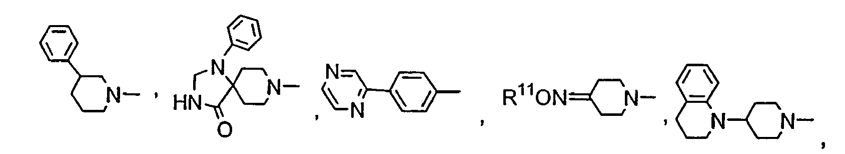 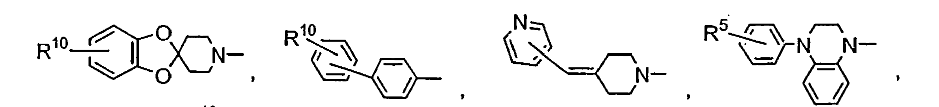 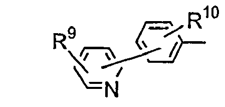 or an N-oxide thereof, 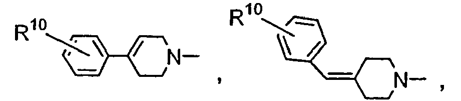 or Y and Z together form a piperidinyl or pyrrolidinyl ring fused to a monocyclic or bicyclic aryl or a monocyclic or bicyclic heteroaryl ring wherein X is attached to the N atom of the piperidinyl or pyrrolidinyl ring;
R¹ is 1 to 3 substituents independently selected from hydrogen, C₁-C₆-alkyl, -CF₃, halogen, -NO₂, -NR¹²R¹³, C₁-C₆ alkoxy, C₁-C₆ alkylthio, C₁-C₆ alkylsulfinyl, C₁-C₆ alkylsulfonyl, -COOR⁷ or -C(O)NR²R³;
R² and R³ are independently selected from the group consisting of hydrogen and C₁-C₆ alkyl;
m and n are independently 2-3;
p and q are independently 0-2;
Q and Q¹ are independently selected from the group consisting of 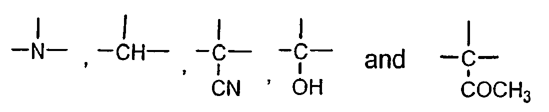 provided that at least one of Q and Q¹ is 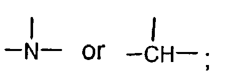 R⁴ is 1-2 substituents independently selected from the group consisting of hydrogen, C₁-C₆alkyl, R¹-aryl and R¹-heteroaryl, or two R⁴ substituents on the same carbon can form =O;
R⁵ is 1 to 5 substituents independently selected from the group consisting of hydrogen, halogen, C₁-C₆ alkyl, hydroxy, C₁-C₆ alkoxy, -CN, di-((C₁-C₆)alkyl)amino, -CF₃, -OCF₃, acetyl, -NO₂, hydroxy(C₁-C₆)alkoxy, (C₁-C₆)-alkoxy(C₁-C₆)alkoxy, di-((C₁-C₆)-alkoxy)(C₁-C₆)alkoxy, (C₁-C₆)-alkoxy(C₁-C₆)alkoxy-(C₁-C₆)-alkoxy, carboxy(C₁-C₆)-alkoxy, (C₁-C₆)-alkoxycarbonyl(C₁-C₆)alkoxy, (C₃-C₆)cycloalkyl(C₁-C₆)alkoxy, di-((C₁-C₆)alkyl)amino(C₁-C₆)alkoxy, morpholinyl, (C₁-C₆)alkyl-SO₂-, (C₁-C₆)alkyl-SO₂-(C₁-C₆)alkoxy, tetrahydropyranyloxy, (C₁-C₆)alkylcarbonyl(C₁-C₆)-alkoxy, (C₁-C₆)-alkoxycarbonyl, (C₁-C₆)alkylcarbonyloxy(C₁-C₆)-alkoxy, -SO₂NH₂, phenoxy, 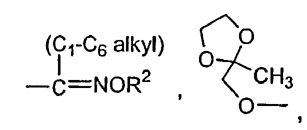 (R²O)₂-P(O)-CH₂-O- and (R²O)₂-P(O)-; or adjacent R⁵ substituents together are -O-CH₂-O-, -O-CH₂CH₂-O-, -O-CF₂-O- or -O-CF₂CF₂-O-and form a ring with the carbon atoms to which they are attached;
R⁶ is (C₁-C₆)alkyl, R⁵-phenyl, R⁵-phenyl(C₁-C₆)alkyl, thienyl, pyridyl, (C₃-C₆)-cycloalkyl, (C₁-C₆)alkyl-OC(O)-NH-(C₁-C₆)alkyl-, di-((C₁-C₆)alkyl)aminomethyl, or 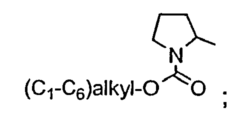 R⁷ is (C₁-C₆)alkyl, R⁵-phenyl or R⁵-phenyl(C₁-C₆)alkyl;
R⁸ is hydrogen or C₁-C₆ alkyl; or R⁷ and R⁸ together are -(CH₂)ₚ-A-(CH₂)_{q}, wherein p and q are independently 2 or 3 and A is a bond, -CH₂-, -S- or -O-, and form a ring with the nitrogen to which they are attached;
R⁹ is 1-2 substituents independently selected from the group consisting of hydrogen, C₁-C₆ alkyl, hydroxy, C₁-C₆ alkoxy, halogen, -CF₃ and (C₁-C₆)alkoxy-(C₁-C₆)alkoxy;
R¹⁰ is 1 to 5 substituents Independently selected from the group consisting of hydrogen; halogen, C₁-C₆ alkyl, hydroxy, C₁-C₆ alkoxy, -CN, -NH₂, C₁-C₆alkylamino, di-((C₁-C₆)alkyl)amino, -CF₃, -OCF₃, -S(O)₀₋₂(C₁-C₆)alkyl and -CH₂-SO₂-phenyl;
R¹¹ is H, C₁-C₆ alkyl, phenyl, benzyl, C₂-C₆ alkenyl, C₁-C₆ alkoxy(C₁-C₆)alkyl, di-((C₁-C₆)alkyl)amino(C₁-C₆)alkyl, pyrrolidinyl(C₁-C₆)alkyl or piperidino(C₁-C₆)alkyl;
R¹² is H or C₁-C₆ alkyl;
R¹³ is H, (C₁-C₆)alkyl-C(O)- or (C₁-C₆)alkyl-SO₂-;
R¹⁴ is H, halogen, C₁-C₆ alkyl, hydroxy(C₁-C₆)alkyl, C₁-C₆ alkoxy(C₁-C₆)alkyl, thio(C₁-C₆)alkyl, (C₁-C₆)alkythlo(C₁-C₆)alkyl or NR²R³-(C₁-C₆)alkyl; and
R¹⁵ is H, halogen, C₁-C₆ alkyl or C₁-C₆ alkoxy.

Exemplary compounds of formula V are those wherein R is R¹-furanyl, R¹-thienyl, R¹-pyrrolyl, R¹-pyridyl or R¹⁰-phenyl, more preferably R¹-furanyl or R¹⁰-phenyl. R¹ is preferably hydrogen or halogen. R¹⁰ is preferably hydrogen, halogen, alkyl or -CF₃. Another group of preferred compounds is that wherein X is alkylene, preferably ethylene. Y is preferably 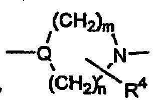 wherein Q is 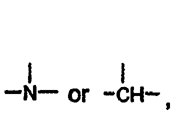 with Q preferably being nitrogen. Preferably, m and n are each 2, and R⁴ is H. A preferred definition for Z is R⁵-phenyl or R⁵-heteroaryl. R⁵ is preferably H, halogen, alkyl, alkoxy, hydroxyalkoxy or alkoxyalkoxy. R⁶ is preferably R⁵-phenyl.

Exemplary specific compounds of formula V are those of the formula VA 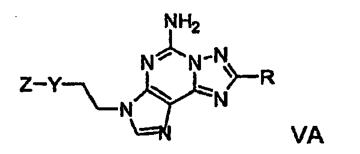 wherein R and Z-Y are as defined in the following table:

| Z-Y- | R |
|---|---|
| 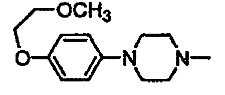 | 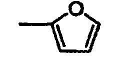 |
| 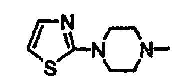 | 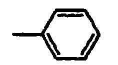 |
| 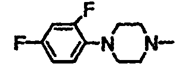 | 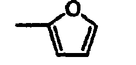 |
| 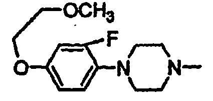 | 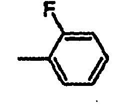 |
| 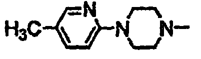 | 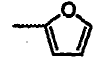 |
| 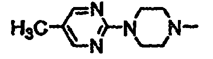 | 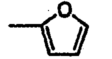 |
| 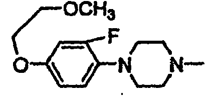 | 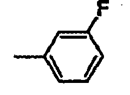 |
| 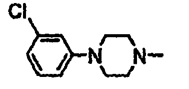 | 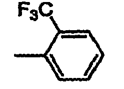 |
| 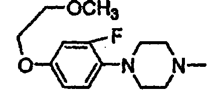 | 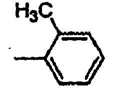 |
| 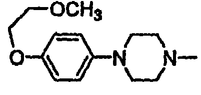 | 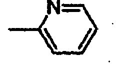 |

WO 03/048165 discloses adenosine A₂ₐ receptor antagonist compounds having the structural formula VI 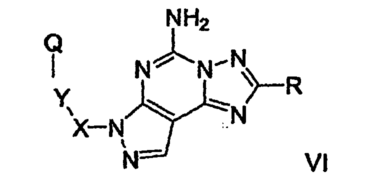 or a pharmaceutically acceptable salt or solvate of said compound, wherein:
R is selected from the group consisting of R¹-furanyl-, R¹-thienyl-, R¹-pyridyl-, R¹-oxazolyl-, R¹-pyrrolyl- and R²-aryl-;
X is -(CH₂)ₙ-;
Y is a piperidinyl, pyrrolidinyl or azepanyl group with an aryl or heteroaryl moiety fused to two adjacent carbon atoms on Y, wherein X is attached to the N atom of the piperidinyl, pyrrolidinyl or azepanyl group;
Q is 1-4 substituents, which can be the same or different, and are independently selected from the group consisting of hydrogen, cycloalkyl, cycloheteroalkyl, amino, aryl, aralkyl, heteroaryl, alkyl, CF₃, CN, halogen, NO₂, alkoxy, alkoxyalkoxy, cycloalkylalkoxy, acyloxy, alkylamino, acylamino, alkylsulfonamino, alkylaminosulfonyl, dialkylaminosulfonyl, NH₂SO₂-, and hydroxy;
n is 1 to 4;
R¹ is 1-3 substituents, which may be the same or different, and are independently selected from the group consisting of hydrogen, alkyl, CF₃, halogen and NO₂; and
R² is 1-3 substituents, which may be the same or different, and are independently selected from the group consisting of hydrogen, alkyl, CF₃, halogen, NO₂, alkoxy, acyloxy, alkylamino, acylamino, alkylsulfonamido, alkylaminosulfonyl, dialkylaminosulfonyl, aminosulfonyl, and hydroxyl.

In exemplary compounds of formula VI, Y is 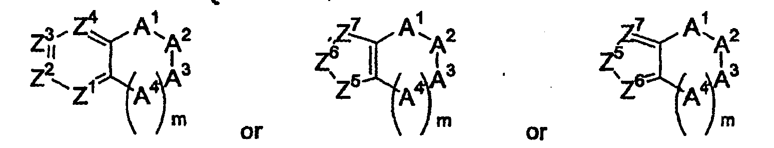 wherein A¹ is N-X, and A² and A³ each are CR⁴R⁵, or
A¹ and A³ each are CR⁴R⁵, and A² is N-X, or
A¹ and A² each are CR⁴R⁵, and A³ is N-X;
A⁴ is CR⁴R⁵;
Z¹, Z², Z³ and Z⁴ , which can the same or different, are each independently selected from the group consisting of N and CR³, provided that 0-2 of Z¹, Z², Z³ or Z⁴ are N and the remainder are CR³;
Z⁵ is NR⁵, O, S or CR⁴R⁵;
Z⁶ is N or CR³;
Z⁷ is N or CR³;
m is an integer from 0 to 2;
R³ is selected from the group consisting of hydrogen, cycloalkyl, amino, aryl, heteroaryl, C₁-C₆-alkyl, CF₃, CN, halogen, NO₂, C₁-C₆-alkoxy, C₁-C₆-acyloxy, C₁-C₆-alkylamino, C₁-C₆-acylamino, C₁-C₆-alkylsulfonamino, C₁-C₆-alkylaminosulfonyl, C₁-C₆-dialkylaminosulfonyl, NH₂-SO₂-, and hydroxy;
R⁴ is selected from the group consisting of hydrogen, hydroxyalkyl, aryl, aralkyl, C₁-C₆-alkyl, C₁-C₆-alkoxy, CF₃, CN, halogen, hydroxy, and NO₂; and
R⁵ is hydrogen or C₁-C₆ alkyl.

Exemplary specific examples of compounds of formula VI include compounds of the formula:

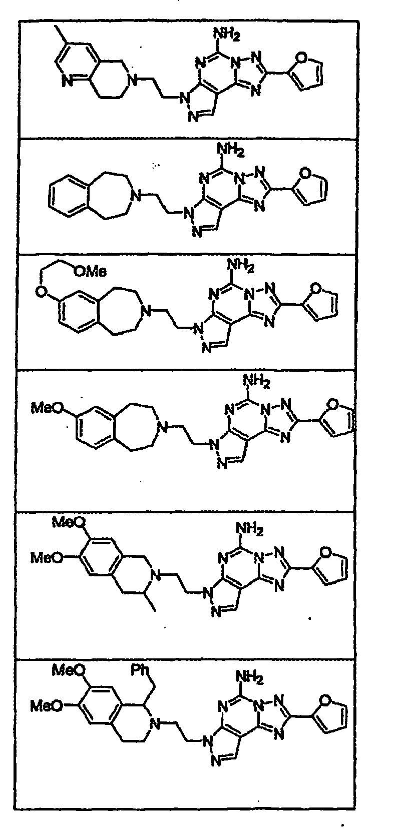

WO 03/048164 discloses adenosine A₂ₐ receptor antagonist compounds having the structural formula VII 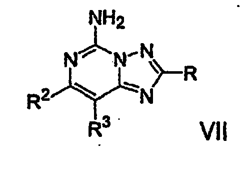 or a pharmaceutically acceptable salt or solvate thereof; wherein:
R is selected from the group consisting of R⁴-heteroaryl, R⁵-phenyl, (C₄-C₆)cycloalkenyl, -C(=CH₂)CH₃, -C≡C-CH₃, 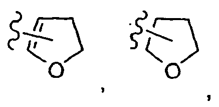 -CH=C(CH₃)₂, 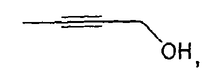 and -CH=CH-CH₃;
R² is selected from the group consisting of -W-X, -NR¹⁹(CH₂)ₘ-W-X, and - NR¹⁹CH(CH₃)-W-X, or
R² is selected from the group consisting of alkyl, alkenyl and -NR¹⁸R¹⁹, wherein said alkyl, alkenyl or -NR¹⁸R¹⁹ is optionally substituted by -W-X;
R³ is selected from the group consisting of H, halo, alkyl, trifluoromethyl, alkoxy, alkoxyalkyl, hydroxyalkyl, alkylamino, alkylaminoalkyl, dialkylamino, dialkylaminoalkyl, aminoalkyl, aryl, heteroaryl, and CN;
R⁴ is 1 to 3 substituents, which can be the same or different, and are independently selected from the group consisting of hydrogen, (C₁-C₆)-alkyl, -CF₃, halogen, -NO₂, -NR¹⁵R¹⁶, (C₁-C₆)alkoxy, (C₁-C₆)alkylthio, (C₁-C₆)alkylsulfinyl, (C₁-C₆)alkylsulfonyl, -COOR¹⁷ and -C(O)NR⁶R⁷;
R⁵ is 1 to 5 substituents, which can be the same or different, and are independently selected from the group consisting of hydrogen, halogen, (C₁-C₆)alkyl, hydroxy, (C₁-C₆)alkoxy, -CN, -NH₂, (C₁-C₆)alkylamino, di-((C₁-C₆)alkyl)amino, -CF₃, - OCF₃, -S(O)₀₋₂(C₁-C₆)alkyl and -CH₂-SO₂-phenyl;
R⁶ and R⁷, which can be the same or different, are each independently selected from the group consisting of hydrogen and (C₁-C₆)alkyl;
R⁸ is 1 to 5 substituents, which can be the same or different, and are independently selected from the group consisting of hydrogen, halogen, (C₁-C₆)alkyl, hydroxy, C₁-C₆ alkoxy, -CN, amino, di-((C₁-C₆)alkyl)amino, -CF₃, -OCF₃, acetyl, -NO₂, hydroxy(C₁-C₆)alkoxy, (C₁-C₆)-alkoxy(C₁-C₆)alkoxy, di-((C₁-C₆)-alkoxy)(C₁-C₆)alkoxy, (C₁-C₆)-alkoxy(C₁-C₆)alkoxy-(C₁-C₆)-alkoxy, carboxy(C₁-C₆)-alkoxy, (C₁-C₆)-alkoxycarbonyl(C₁-C₆)alkoxy, (C₃-C₆)cycloalkyl(C₁-C₆)alkoxy, di-((C₁-C₆)alkyl)amino(C₁-C₆)alkoxy, morpholinyl, (C₁-C₆)alkyl-SO₂-, (C₁-C₆)alkyl-SO₂-(C₁-C₆)alkoxy, tetrahydropyranyloxy, (C₁-C₆)alkylcarbonyl(C₁-C₆)-alkoxy, (C₁-C₆)-alkoxycarbonyl, (C₁-C₆)alkylcarbonyloxy(C₁-C₆)-alkoxy, -SO₂NH₂, phenoxy, 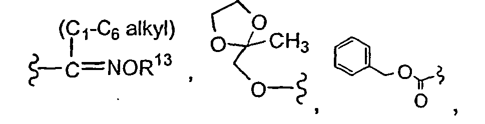 -O-CH₂-P(O)(OR⁶)₂,- and -P(O)(OR⁶)₂; or
adjacent R⁸ substituents together are -O-CH₂-O-, -O-CH₂CH₂-O-, -O-CF₂-O-or
-O-CF₂CF₂-O- and form a ring with the carbon atoms to which they are attached;
R⁹ is selected from the group consisting of (C₁-C₆)alkyl, R⁸-aryl-, R⁸-aryl(C₁-C₆)alkyl-, thienyl, pyridyl, (C₃-C₆)-cycloalkyl, (C₁-C₆)alkyl-OC(O)-NH-(C₁-C₆)alkyl-, di-((C₁-C₆)alkyl)aminomethyl, cycloheteroalkyl(C₁-C₆)alkyl, aryloxy(C₁-C₆)alkyl, alkoxy(C₁-C₆)alkyl and 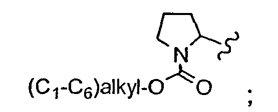
R¹⁰ is 1-2 substituents, which can be the same or different, and are independently selected from the group consisting of hydrogen, (C₁-C₆)alkyl, R⁵-aryl and R⁴-heteroaryl, or two R¹⁰ substituents on the same carbon can form =O;
R¹¹ is hydrogen or (C₁-C₆)alkyl; -C(O)alkyl, or R¹⁷ and R¹¹ taken together are -(CH₂)ₚ-A-(CH₂)_{q}, wherein p and q are each independently 2 or 3 and A is selected from the group consisting of a bond, -CH₂-, -S- and -O-, and form a ring with the nitrogen to which they are attached;
R¹² is 1-2 substituents, which can be the same or different, and are independently selected from the group consisting of hydrogen, (C₁-C₆)alkyl, hydroxy, (C₁-C₆)alkoxy, halogen, and -CF₃;
R¹³ is selected from the group consisting of H, (C₁-C₆)alkyl, phenyl, benzyl, (C₂-C₆)alkenyl, (C₁-C₆)alkoxy(C₁-C₆)alkyl, di-((C₁-C₆)alkyl)amino(C₁-C₆)alkyl, pyrrolidinyl(C₁-C₆)alkyl and piperidino(C₁-C₆)alkyl;
R¹⁴ is selected from the group consisting of H, halogen, (C₁-C₆)alkyl or (C₁-C₆)alkoxy;
R¹⁵ is selected from the group consisting of H and (C₁-C₆)alkyl;
R¹⁶ is selected from the group consisting of H, (C₁-C₆)alkyl-C(O)- and (C₁-C₆)alkyl-SO₂-;
R¹⁷ is selected from the group consisting of (C₁-C₆)alkyl, (C₁-C₆)hydroxyalkyl, (C₃-C₆)cycloalkyl, (C₁-C₆)alkoxy(C₁-C₆)alkoxy, (C₁-C₆)alkoxy, (C₁-C₆)alkoxy(C₁-C₆)alkyl, allyl, propargyl, R⁸-heteroaryl-, R⁸-aryl- and R⁸-aryl(C₁-C₆)alkyl-;
R¹⁸ is selected from the group consisting of a bond, -CH₂-, -CH(OH)-, -CH(CH₃)-, -C(CH₃)ₙ-, -(CH₂)ₙ-, and -O(CH₂)ₙ-,
R¹⁹ is selected from the group consisting of H, (C₁-C₆)alkyl, (C₁-C₆)alkyl(C₁-C₆)cycloalkyl, (C₁-C₆)cycloalkyl(C₁-C₆)alkyl and (C₁-C₆)alkoxy(C₁-C₆)alkyl;
Q and Q¹ can be the same or different and are each independently selected from the group consisting of 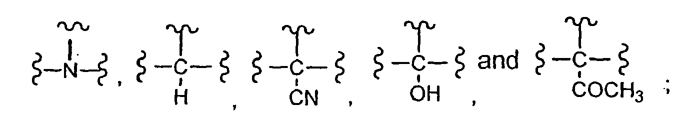
m and n are each independently 1-3;
p and q are each independently 0-2;
s is 0-4;
W is aryl or heteroaryl having 1-3 heteroatoms, which can be the same or different, and are independently selected from the group consisting of N, O and S, and wherein said aryl or heteroaryl is optionally substituted with 1-3 substituents, which can be the same or different, and are independently selected from the group consisting of alkyl, aryl, alkylcycloalkyl, halo, hydroxy, hydroxyalkyl, alkoxy, alkylalkoxy, alkoxyalkoxy, -NR⁶R⁷, (C₂-C₆)alkene, and -CN, or
X is selected from the group consisting of H, NH₂, -N(R⁶)(CH₂)ₛ-aryl, - N(R⁶)(CH₂)ₛ-heteroaryl, -N(R⁶)(CH₂)ₘ₊₁-OH, and -N(CH₃)₂, or
X is -R¹⁸-Y-Z;
Y is selected from the group consisting of -N(R⁶)CH₂CH₂N(R⁷)-, -N(R⁶)(CH₂)ₙaryl, -OCH₂CH₂N(R⁶)-, -O-, -S-, -CH₂S-, -(CH₂)₂₋₃-N(R⁶)-, R⁸-divalent heteroaryl, 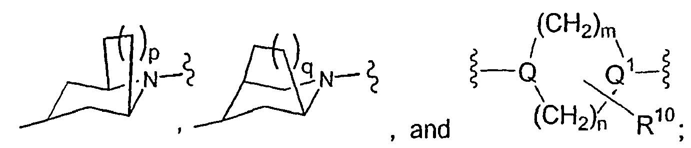 and
Z is selected from the group consisting of H, alkyl, alkoxyalkyl, R⁸-aryl-, R⁸-aryl(C₁-C₆)alkyl-, R⁸-heteroaryl-, R⁸-bicyclicalkyl-, aminoalkyl, alkylamino, NH₂, -N-(R⁶)(CH₂)ₛ-aryl, -N(R⁶)(CH₂)ₛ-heteroaryl, -N(R₆)C(O)OR¹⁷, alkylcycloheteroalkyl, cycloheteroalkyl, cycloheteroalkylalkyl, alkoxycycloheteroalkyl, heteroaryl; R⁸-benzofused heteroaryl-, diphenylmethyl and R⁹-C(O)-; or
   when Y is 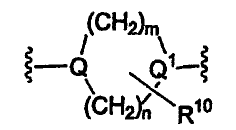 Z can also be -OH, R⁹-SO₂-, R¹⁷-N(R¹¹)(CH₂)ₛ-C(O)-, R¹⁷-OC(O)-, R¹⁷-O(CH₂)ₙC(O)-, benzofused heteroaryl(CH₂)ₙC(O)-, benzofused heteroaryl(CH₂)ₙ- or R¹⁷-N(R¹¹)-C(S)-; or
when Q is 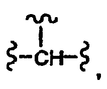 Z can also be R¹⁷R¹¹N-, phenylamino or pyridylamino; or
Z and Y taken together are selected from the group consisting of 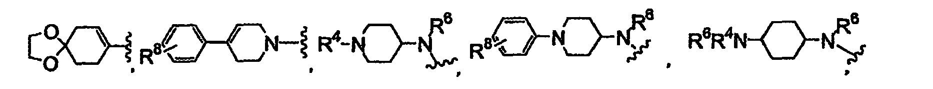 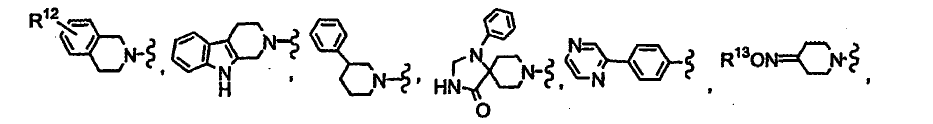 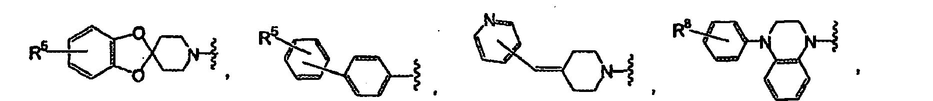 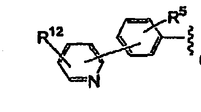 or an N-oxide thereof,

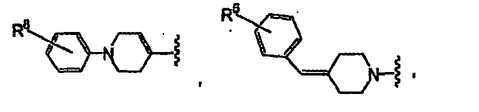

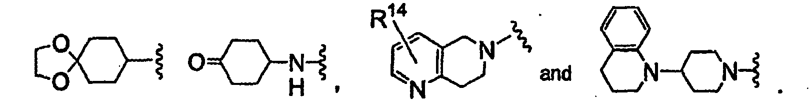

Exemplary compounds of formula VII are those having the following structures:

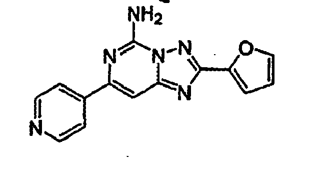

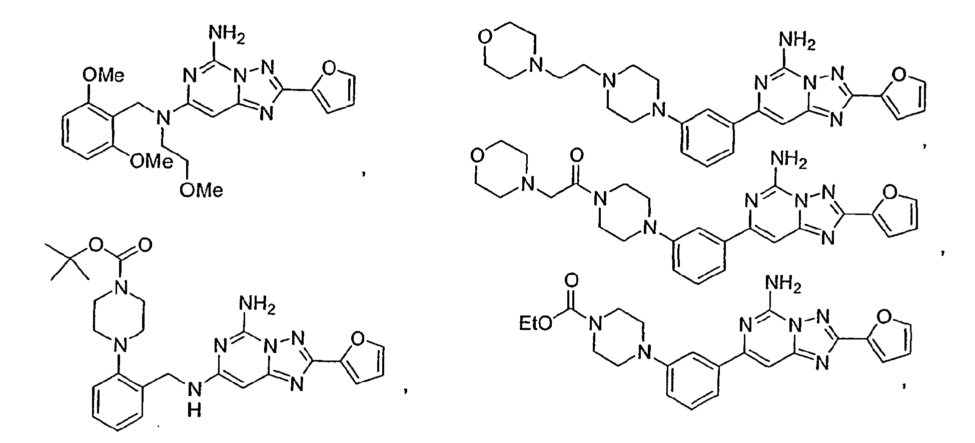

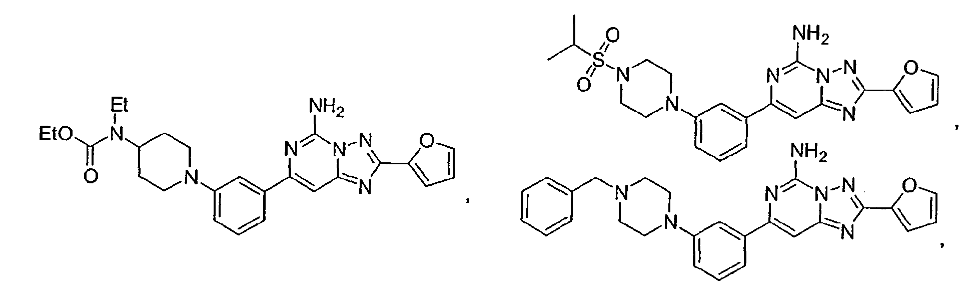

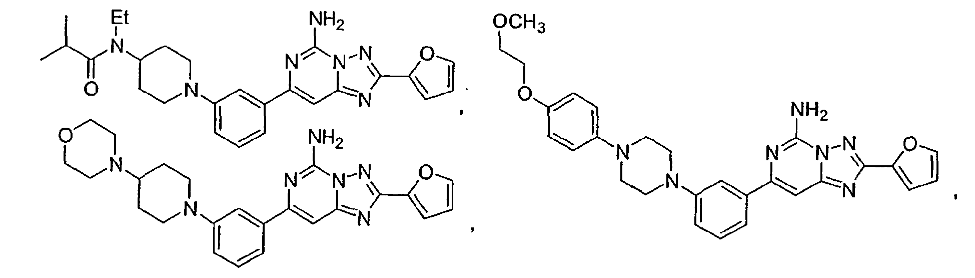

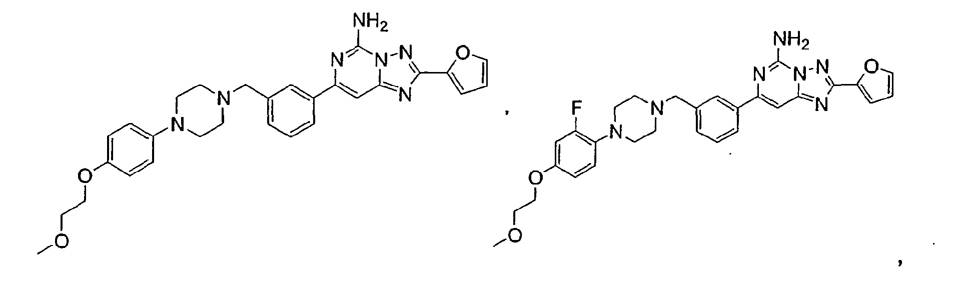

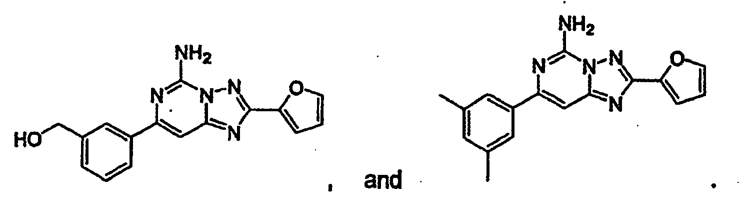

WO 03/048163 discloses adenosine A₂ₐ receptor antagonist compounds having the structural formula VIII 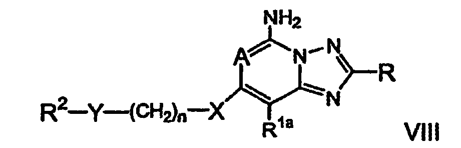 or a pharmaceutically acceptable salt thereof, wherein: .
A is C(R¹) or N;
R¹ and R^{1a} are independently selected from the group consisting of H, (C₁-C₆)-alkyl, halo, CN and -CF₃;
Y is -O-, -S-, -SO-, -SO₂-, R⁵-heteroaryldiyl, R⁵-arylene or 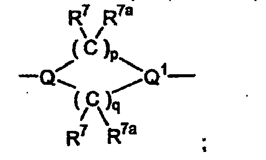
p and q are independently 2-3;
Q and Q¹ are independently selected from the group consisting of 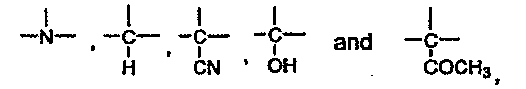 provided that at least one of Q and Q¹ is 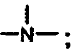
R is R⁵⁻aryl, R⁵⁻heteroaryl, R⁶-(C₂-C₆)alkenyl or R⁶-(C₂-C₆)alkynyl;
R² is R⁵⁻aryl, R⁵⁻heteroaryl, R⁵⁻aryl(C₁-C₆)alkyl or R⁵⁻heteroaryl(C₁-C₆)alkyl; or R²-Y is 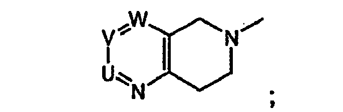
U, V, and W are independently selected from the group consisting of N and CR¹, provided that at least one of U, V and W is CR¹;
n is 1, 2 or 3; and
   (a) A is C(R¹) and X is -C(R³)(R^{3a})-, -C(O)-, -O-, -S-, -SO-, -SO₂-, R⁴-arylene, R⁴-heteroaryldiyl, or -N(R⁹)-; or A is C(R¹), Y is a bond, and X is -C(R³)(R^{3a})-, -C(O)-, -O-, -S-, -SO-, -SO₂-, R⁴-arylene, -N(R⁹)- or R⁴-heteroaryldiyl, provided that when X is -N(R⁹)- or R⁴-heteroaryldiyl, R² is not phenyl or phenyl-(C₁-C₆)alkyl; or
   (b) A is N, X is -N(R⁹)-, Y is R⁵-arylene and R² is 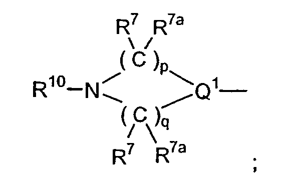 or n is 2 or 3; and
   (c) A is N and X is -C(R³)(R^{3a})-, -C(O)-, -O-, -S-, -SO-, -SO₂-, -N(R⁹)-, R⁴-arylene or R⁴-heteroaryldiyl; or A is N, Y is a bond and X is -C(O)-, -N(R⁹)-, R⁴-arylene or R⁴-heteroaryldiyl; or A is N, Y is -N(R^{9a})-, -C(O)N(R^{9a})- or -O-(CH₂)₂-N(R^{9a})-, and X is -N(R⁹)-; or A is N, X is -N(R⁹)-, and Y and R² together are 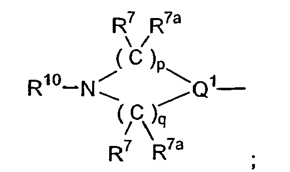 or n is 0; and
   (d) A is N, Y is a bond, X is -N(R⁹)-, and R² is 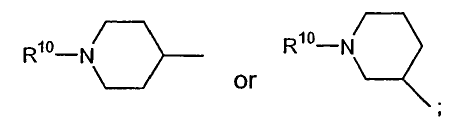 or
   (e) A is N, X is -N(R⁹)- and Y and R² together are 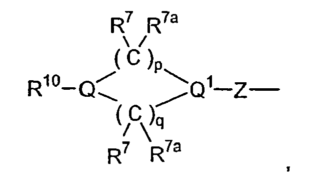 wherein Z is -C(O)-CH₂-, -C(O)-CH(C₁-C₆ alkyl)-, -CH₂-CH(C₁-C₆ alkyl)-, or -CH(C₁-C₆ alkyl)-CH₂-;
R³ and R^{3a} are independently selected from the group consisting of H, -OH, C₁-C₆ alkyl, hydroxy(C₁-C₆)alkyl, (C₁-C₆)alkoxy(C₁-C₆)alkyl, amino(C₁-C₆)alkyl, (C₁-C₆)alkylamino(C₁-C₆)alkyl and di(C₁-C₈)alkylamino(C₁-C₆)alkyl;
R⁴ is 1-3 substituents selected from the group consisting of H, (C₁-C₆)alkyl, -OH, (C₁-C₆)alkoxy, (C₁-C₆)alkoxy(C₁-C₆)alkoxy, halo, -CF₃, and -CN;
R⁵ is 1-3 substituents independently selected from the group consisting of H, (C₁-C₆)alkyl, -OH, (C₁-C₆)alkoxy, (C₁-C₆)alkoxy(C₁-C₆)alkyl, (C₁-C₆)alkoxy(C₁-C₆)-alkoxy, halo, -CF₃, -CN, -NH₂, (C₁-C₆)alkylamino, di(C₁-C₆)alkylamino, amino(C₁-C₆)-alkyl, (C₁-C₆)alkylamino(C₁-C₆)alkyl, di(C₁-C₆)alkylamino(C₁-C₆)alkyl, (C₁-C₆)alkanoylamino, (C₁-C₆)alkanesulfonylamino, (C₁-C₆)alkylthio, (C₁-C₆)alkylthio(C₁-C₆)alkyl, R⁶-(C₂-C₆)alkenyl, R⁶-(C₂-C₆)alkynyl, hydroxy(C₁-C₆)alkyl, (C₁-C₆)alkoxy-C(O)-amino, or heterocycloalkyl(C₁-C₆)alkyl;
R⁶ is 1 to 3 substituents independently selected from the group consisting of H, -OH, (C₁-C₆)alkoxy and halo;
R⁷ and R^{7a} are independently selected from the group consisting of H, (C₁-C₆)alkyl, (C₁-C₆)alkoxy(C₁-C₆)alkyl, R⁸-aryl and R⁸-heteroaryl, or an R⁷ and an R^{7a} substituent on the same carbon can form =O;
R⁸ is 1 to 3 substituents independently selected from H, (C₁-C₆)alkyl, -OH, (C₁-C₆)alkoxy, (C₁-C₆)alkoxy(C₁-C₆)alkoxy, halo, -CF₃, and -CN;
R⁹ and R^{9a} are independently selected from the group consisting of H, (C₁-C₆)alkyl, hydroxy(C₂-C₆)alkyl, (C₁-C₆)alkoxy(C₂-C₆)alkyl, amino(C₂-C₆)alkyl, (C₁-C₆)alkylamino(C₂-C₆)alkyl, di(C₁-C₆)alkylamino(C₂-C₆)alkyl, halo-(C₃-C₆)alkenyl, CF₃-(C₁-C₆)alkyl, (C₃-C₆)alkenyl, (C₃-C₆)cycloalkyl and (C₃-C₆)cycloalkyl-(C₁-C₆)alkyl; and
R¹⁰ is H, -C(O)-O-(C₁-C₆)alkyl, R⁵-aryl, -C(O)-(C₁-C₆)alkyl, -C(O)-(R⁵-aryl) or R⁵-aryl-(C₁-C₆)alkyl.

Exemplary compounds of formula VIII are those wherein A is N. R is preferably furyl. R^{1a} is preferably hydrogen. Another group of preferred compounds is that wherein X is -O-, -S-, -N(R⁹)- or R⁴-arylene, with compounds wherein X is -N(R⁹)- being more preferred. R⁹ is preferably C₁-C₆ alkyl. Preferred definitions for Y are a bond or piperazinyl. R² is preferably R⁵-aryl. When Y and/or R² is 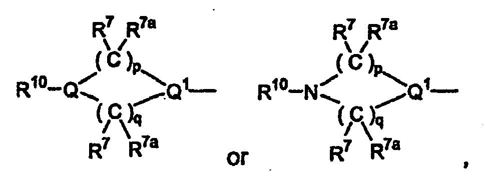 Q is preferably N, Q¹ is preferably N, p and q are each preferably 2, each R⁷ and R^{7a} is preferably hydrogen, and R¹⁰ is preferably -C(O)-O-(C₁-C₆)alkyl, -C(O)-(C₁-C₆)alkyl or -C(O)-(R⁵-aryl). R⁵ is preferably 1 or 2 substituents selected from the group consisting of H, (C₁-C₆)alkoxy, (C₁-C₆)alkoxy(C₁-C₆)-alkoxy, halo and -CF₃. R⁴ is preferably H, halo or (C₁-C₆)alkyl. R³ and R^{3a} are preferably independently selected from H and (C₁-C₆)alkyl. R^{9a} is preferably H or (C₁-C₆)alkyl. R⁶ is preferably hydrogen. Exemplary specific examples of compounds of formula VIII include compounds of the formula 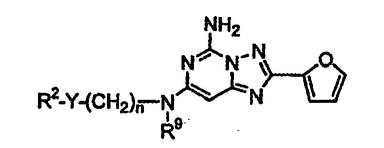 wherein R²-Y-(CH₂)ₙ-N(R⁹)- is as defined in the table:

| R²-Y-(CH₂)ₙ-N(R⁹)- |
|---|
| 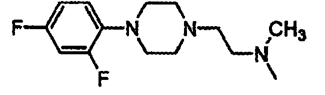 |
| 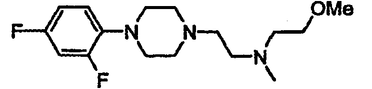 |
| 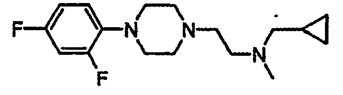 |
| 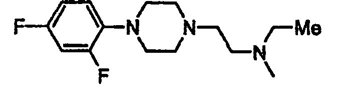 |
| 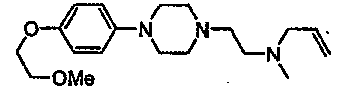 |
| 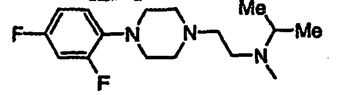 |
| 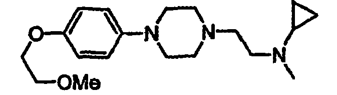 |
| 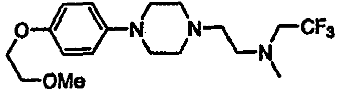 |
| 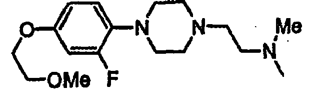 |
| 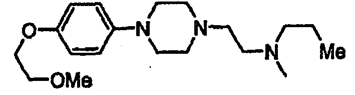 |

WO 01/02409 discloses adenosine A₂ₐ receptor antagonist compounds having the structural formula IX 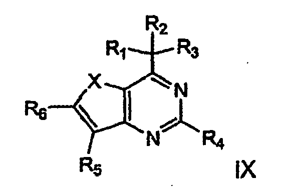 wherein
X is O or S;
R₁ and R₂ are independently selected from hydrogen, alkyl, aryl, hydroxy, alkoky, aryloxy, cyano, nitro, CO₂R₇, COR₇, OCOR₇, CONR₇R₈, CONR₇NR₈R₉, OCONR₇R₈, NR₇R₈, NR₇COR₈, NR₇CONR₈R₉, NR₇CO₂R₈, NR₇SO₂R₈, NR₇CONR₈NR₉R₁₀, NR₇NR₈CO₂R₉, NR₇NR₈CONR₉R₁₀, NR₇SO₂NR₈R₉, SO₂R₇, SOR₇, SR₇ and SO₂NR₇R₈, or R₁and R₂ together form a carbonyl group (C=O), an oxime group (C=NOR₁₁), an imine group (C=NR₁₁) or a hydrazine group (C=NNR₁₁R₁₂), or R₁and R₂ together form a 5, 6 or 7 membered carbocyclic or heterocyclic ring;
R₃ is alkyl or aryl;
R₄, R₅ and R₆ ate independently selected from hydrogen, alkyl, aryl, halogen, hydroxy, nitro, cyano, alkoxy, aryloxy, CO₂R₇, COR₇, OCOR₇, SO₂R₇, SOR₇, SR₇, SO₂NR₇R₈, , CONR₇R₈, CONR₇NR₈R₉, OCONR₇R₈, NR₇R₈, NR₇COR₈, NR₇CONR₈R₉, NR₇CO₂R₈, NR₇SO₂R₈, CR₇=NOR₈, NR₇CONR₈NR₉R₁₀, NR₇NR₈CO₂R₉, NR₇NR₈CONR₉R₁₀, SO₂NR₇NR₈R₉, NR₇SO₂NR₈R₉, NR₇NR₈SO₂R₉, NR₇NR₈CO₂R₉, NR₇NR₈R₉ and NR₇CSNR₈R₉, or R₅ and R₆ together form a 5, 6 or 7 membered carbocyclic or heterocyclic ring; and
R₇, R₈, R₉, R₁₀, R₁₁ and R₁₂ are independently selected from hydrogen, alkyl and aryl, or a pharmaceutically acceptable salt or prodrug thereof.

The adenosine A₂ₐ receptor antagonists are prepared by known methods as described in the cited patents and applications.

As used herein, "patient" means a mammal, especially a human.

It is contemplated that more than one adenosine A₂ₐ receptor antagonist (e.g., 2 or 3) can be administered to treat EPS, dystonia, RLS or PLMS; preferably, one adenosine A₂ₐ receptor antagonist is administered.

Antipsychotic agents causing the EPS treated by adenosine A₂ₐ receptor antagonists and for use in combination with adenosine A₂ₐ receptor antagonists include typical and atypical antipsychotic agents. Typical antipsychotics include loxapine, haloperidol, chlorpromazine, prochlorperazine and thiothixene. Atypical antipsychotics include clozapine, olanzapine, loxapine, quetlapine, ziprasidone and risperidone.

Tricyclic antidepressants causing dystonia treated by adenosine A₂ₐ receptor antagonists include perphenazine, amitriptyline, desipramine, doxepin, trimipramine and protriptyline. Anticonvulsants which may cause dystonia, but which also may be useful in treating ERLS or PLMS include phenytoin, carbamazepine and gabapentin.

Dopamine agonists useful in treating RLS and PLMS include pergolide, pramipexole, ropinerole, fenoldopam and cabergoline.

Opioids useful in treating PRLS and PLMS include codeine, hydrocodone, oxycodone, propoxyphene and tramadol.

Benzodiazepines useful in treating PRLS and PLMS include donazepam, triazolam and temazepam.

The antipsychotics, tricyclic antidepressants, anticonvulsants, dopamine agonists, opioids and benzodiazepines are commercially available and are described in the literature, e.g., in The Physicians' Desk Reference (Montvale: Medical Economics Co., Inc., 2001).

It is contemplated that two or more A₂ₐ receptor antagonists could be administered in combination with one or more other agents (e.g., antipsychotics, tricyclic antidepressants, anticonvulsants, dopamine agonists, opioids or benzodiazepines), although administration of one A₂ₐ antagonist in combination with one other agent is preferred for each of the indications. While administration of separate dosage forms of the A₂ₐ antagonist(s) and the other agent(s) are preferred, it is also contemplated that the other agent(s) could be combined in a single dosage form with the A₂ₐ receptor antagonist(s) for the treatment or prevention of EPS, dystonia, RLS or PLMS.

The adenosine A2a antagonists useful in the present invention are those described in US 6,630,475. A particularly preferred compound for use in the invention is Compound A of the formula 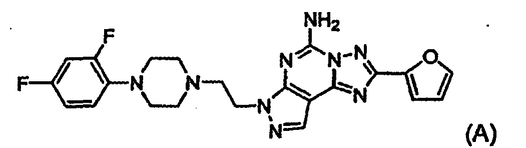 or a pharmaceutically acceptable salt or solvate thereof, disclosed In US 6,630,475 and listed as the first compound in the table of compounds of structure I.

Compounds useful in the invention will show utility as adenosine A₂ₐ receptor antagonists in these assays.
Human Adenosine A₂ₐ and A₁ Receptor Competition Binding Assay Protocol
Membrane sources: A₂ₐ: Human A₂ₐ Adenosine Receptor membranes, Catalog #RB-HA2a, Receptor Biology, Inc., Beltsville, MD. Dilute to 17 µg/100 µl in membrane dilution buffer (see below).
Assay Buffers: Membrane dilution buffer: Dulbecco's Phosphate Buffered Saline (Gibco/BRL) + 10 mM MgCl₂.
Compound Dilution Buffer: Dulbecco's Phosphate Buffered Saline (Gibco/BRL) + 10 mM MgCl₂ supplemented with 1.6 mg/ml methyl cellulose and 16% DMSO. Prepared fresh daily.
Ligands: A₂ₐ: [3H]-SCH 58261, custom synthesis, AmershamPharmacia Biotech, Piscataway, NJ. Stock is prepared at 1 nM in membrane dilution buffer. Final assay concentration is 0.5 nM.

A₁: [3H]- DPCPX, AmershamPharmacia Biotech, Piscataway, NJ. Stock is prepared at 2 nM in membrane dilution buffer. Final assay concentration is 1 nM.

### Non-specific Binding:

A₂ₐ: To determine non-specific binding, add 100 nM CGS 15923 (RBI, Natick, MA). Working stock is prepared at 400 nM in compound dilution buffer.

A₁: To determine non-specific binding, add 100 µM NECA (RBI, Natick, MA). Working stock is prepared at 400 µM in compound dilution buffer.

### Compound Dilution:

Prepare 1 mM stock solutions of compounds in 100% DMSO. Dilute in compound dilution buffer. Test at 10 concentrations ranging from 3 µM to 30 pM. Prepare working solutions at 4X final concentration in compound dilution buffer.

### Assay procedure:

Perform assays in deep well 96 well plates. Total assay volume is 200 µl. Add 50 µl compound dilution buffer (total ligand binding) or 50 µl CGS 15923 working solution (A₂ₐ non-specific binding) or 50 µl NECA working solution (A₁ non-specific binding) or 50 µl of drug working solution. Add 50 µl ligand stock ([3H]-SCH 58261 for A₂ₐ, [3H]- DPCPX for A₁). Add 100 µl of diluted membranes containing the appropriate receptor. Mix. Incubate at room temperature for 90 minutes. Harvest using a Brandel cell harvester onto Packard GF/B filter plates. Add 45 µl Microscint 20 (Packard), and count using the Packard TopCount Microscintillation Counter. Determine IC₅₀ values by fitting the displacement curves using an iterative curve fitting program (Excel). Determine Ki values using the Cheng-Prusoff equation.

### Haloperidol-induced catalepsy in the rat

Male Sprague-Dawley rats (Charles River, Calco, Italy) weighing 175-200 g are used. The cataleptic state is induced by the subcutaneous administration of the dopamine receptor antagonist haloperidol (1 mg/kg, sc), 90 min before testing the animals on the vertical grid test. For this test, the rats are placed on the wire mesh cover of a 25x43 plexiglas cage placed at an angle of about 70 degrees with the bench table. The rat is placed on the grid with all four legs abducted and extended ("frog posture"). The use of such an unnatural posture is essential for the specificity of this test for catalepsy. The time span from placement of the paws until the first complete removal of one paw (*descent latency*) is measured maximally for 120 sec.

The selective A_{2A} adenosine antagonists under evaluation are administered orally at doses ranging between 0.03 and 3 mg/kg, 1 and 4 h before scoring the animals.

In separate experiments, the anti-cataleptic effects were determined for the reference compound, L-DOPA (25, 50 and 100 mg/kg, ip),

For preparing pharmaceutical compositions from the compounds useful in this invention, inert, pharmaceutically acceptable carriers can be either solid or liquid. Solid form preparations include powders, tablets, dispersible granules, capsules, cachets and suppositories. The powders and tablets may be comprised of from about 0.1 to about 99 percent active ingredient. Suitable solid carriers are known in the art, e.g. magnesium carbonate, magnesium stearate, talc, sugar, lactose. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parenteral injection.

Liquid form preparations may also include solutions for intranasal administration.

Aerosol preparations suitable for inhalation may include solutions and solids in powder form, which may be in combination with a pharmaceutically acceptable carrier, such as an inert compressed gas.

Also included are solid form preparations which are intended to be converted, shortly before use, to liquid form preparations for either oral or parenteral administration. Such liquid forms include solutions, suspensions and emulsions.

The compounds useful in the invention may also be deliverable transdermally. The transdermal compositions can take the form of creams, lotions, aerosols and/or emulsions and can be included in a transdermal patch of the matrix or reservoir type as are conventional in the art for this purpose.

Preferably the adenosine A₂ₐ receptor antagonist and the antipsychotic are administered orally.

Preferably, the pharmaceutical preparation is in unit dosage form. In such form, the preparation is subdivided into unit doses containing appropriate quantities of the active component, e.g., an effective amount to achieve the desired purpose.

The quantity of adenosine A₂ₐ receptor antagonist in a unit dose of preparation may be varied or adjusted from about 0.1 mg to 1000 mg, more preferably from about 1 mg to 300 mg, according to the particular application.

The actual dosage employed may be varied depending upon the requirements of the patient and the severity of the condition being treated. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The amount and frequency of administration of the adenosine A₂ₐ receptor antagonist useful in the method of the invention will be regulated according to the judgment of the attending clinician considering such factors as age, condition and size of the patient as well as severity of the symptoms being treated. A typical recommended dosage regimen for an adenosine A₂ₐ receptor antagonist is oral administration of about 10 mg to 2000 mg/day preferably 10 to 1000 mg/day, in two to four divided doses to provide relief from the effects of EPS, dystonia, RLS or PLMS. The compounds are non-toxic when administered within this dosage range.

The doses and dosage regimen of the other agents used in combination with the adenosine A₂ₐ receptor antagonists, i.e., the antipsychotics, tricyclcic antidepressants, anticonvulsants, dopamine agonists, benzodiazepines, opioids, lithium or iron, will be determined by the attending clinician in view of the approved doses and dosage regimen in the package insert, taking into consideration the age, sex and condition of the patient and the severity of the disease. When administered in combination, the adenosine A₂ₐ receptor antagonist and the other agent can be administered simultaneously or sequentially. This is particularly useful when the components of the combination are preferably given on different dosing schedules, e.g., one component is administered daily and the other every six hours, or when the preferred pharmaceutical compositions are different, e.g. one is preferably a tablet and one is a capsule. It is therefore advantageous to provide the adenosine A₂ₐ receptor antagonist and the other agent in a kit comprising, in separate containers in a single package, pharmaceutical compositions for use in combination to treat or prevent EPS, dystonia, RLS or PLMS, wherein one container comprises a pharmaceutical composition comprising an effective amount of an adenosine A₂ₐ receptor antagonist in a pharmaceutically acceptable carrier, and wherein a separate container comprises a pharmaceutical composition comprising an effective amount of another agent appropriate to treat the indicated condition.

Those skilled in the art will recognize that a dosage form for one of the components of the combination can be modified to contain both an adenosine A₂ₐ receptor antagonist and another agent, e.g., an adenosine A₂ₐ receptor antagonist and an antipsychotic or an adenosine A₂ₐ receptor antagonist and a dopamine agonist.

The following example shows the use of adenosine A2a antagonists to attenuate the Extra-Pyramidal Syndrome (EPS) displayed in *cebus apella* monkeys sensitized to the dopamine D₂ receptor antagonist, haloperidol.

### Example

A colony of seven *Cebus apella* monkeys that were previously sensitized to the chronic effects of haloperidol, exhibit EPS when administered haloperidol acutely (0.3 mg/kg, p.o.). Compound A was administered orally (p.o.) at doses of 0.3-30 mg/kg, in conjunction with haloperidol. The studies were conducted using a within-subjects design such that each monkey received all 6 treatments (vehicle and 5 doses of Compound A) in a crossover, balanced design. In all the studies, the group of seven monkeys exhibited baseline levels of EPS when dosed with haloperidol.

Compound A produced a dose-dependent reduction in the maximum EPS score (Figure 1A), as well as a dose-dependent delay in the onset of EPS (Figure 1 B). At a dose of 1 mg/kg, Compound A prevented the onset of EPS in one monkey, and delayed the onset of EPS by 1 hr. Compound A, at a dose of 3 mg/kg, prevented the onset of EPS in two monkeys, and delayed the onset of EPS by almost 2 hr in the remaining monkeys. At 10 and 30 mg/kg, Compound A prevented the onset of EPS in three monkeys and delayed the onset of EPS by an average of 2.3-2.9 hr.

Clinical guidelines for the treatment of RLS and PLMS have been established: see A. L. Chesson et al, Sleep, 22, 7 (1999), p. 961-8. Efficacy of adenosine A₂ₐ antagonists in treating RLS and PLMS can be determined by a method analogous to the clinical method described in the literature for pramipexole and ropinerole by Weimerskirch et al, Annals of Pharmacotherapy, 35, 5 (2001), p. 627-30.

## Claims

1. The use of an adenosine A2a receptor antagonist for the preparation of a medicament for the treatment or prevention of Extra-Pyramldal Syndrome (EPS) in a patient treated with an antipsychotic agent that has the side effect of inducing EPS
wherein the adenosine A2a antagonist is a compound of the formula 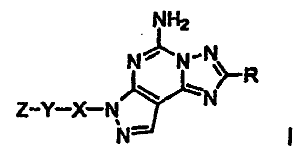 or a pharmaceutically acceptable salt thereof, wherein
R is R¹-furanyl, R¹-thienyl, R¹-pyridyl, R¹-pyridyl N-oxide, R¹-oxazolyl, R¹⁰-phenyl, R¹-pyrrolyl or C₄-C₆ cycloalkenyl;
X is C₂-C₆ alkylene or -C(O)CH₂-;
Y is -N(R²)CH₂CH₂N(R³)-, -OCH₂CH₂N(R²)-, -O-, -S-, -CH₂S-, -(CH₂)₂-NH-, or 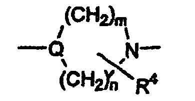 and
Z is R⁵-phenyl, R⁵-phenyl(C₁-C₆)alkyl, R⁵-heteroaryl, diphenylmethyl, R⁶-C(O)-, R⁶-SO₂-, R⁶-OC(O)-, R⁷-N(R⁸)-C(O)-, R⁷-N(R⁸)-C(S)-, 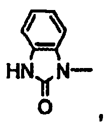 phenyl-CH(OH)-, or phenyl-C(=NOR²)-; or when Q is 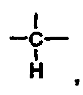 Z is also phenylamino or pyridylamino; or
Z and Y together are 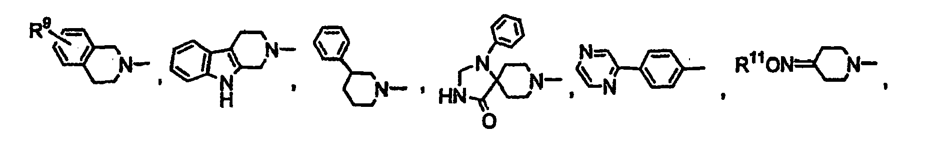 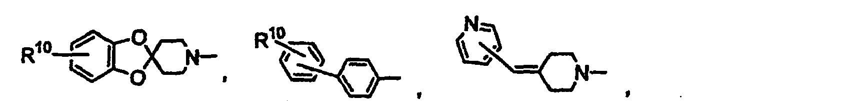 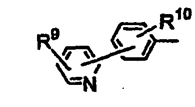 or an N-oxide thereof, 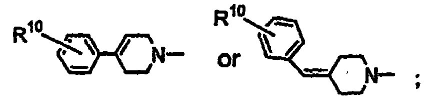 R¹ is 1 to 3 substituents independently selected from hydrogen, C₁-C₆-alkyl, -CF₃, halogen, -NO₂, -NR¹²R¹³, C₁-C₆ alkoxy, C₁-C₆ alkylthio, C₁-C₆ alkylsulfinyl, and C₁-C₆ alkylsulfonyl;
R² and R³ are independently selected from the group consisting of hydrogen and C₁-C₆ alkyl;
m and n are independently 2-3;
Q is 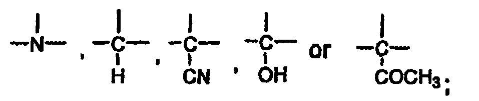 R⁴ is 1-2 substituents independently selected from the group consisting of hydrogen and C₁-C₆alkyl, or two R⁴ substituents on the same carbon can form =O;
R⁵ is 1 to 5 substituents independently selected from the group consisting of hydrogen, halogen, C₁-C₆ alkyl, hydroxy, C₁-C₆ alkoxy, -CN, di-((C₁-C₆)alkyl)amino, -CF₃, -OCF₃, acetyl, -NO₂, hydroxy(C₁-C₆)alkoxy, (C₁-C₆)-alkoxy(C₁-C₆)alkoxy, di-((C₁-C₆)-alkoxy)(C₁-C₆)alkoxy, (C₁-C₆)-alkoxy(C₁-C₆)alkoxy-(C₁-C₆)-alkoxy, carboxy(C₁-C₆)-alkoxy, (C₁-C₆)-alkoxycarbonyl(C₁-C₆)alkoxy, (C₃-C₆)cycloalkyl(C₁-C₆)alkoxy, di-((C₁-C₆)alkyl)amino(C₁-C₆)alkoxy, morpholinyl, (C₁-C₆)alkyl-SO₂-, (C₁-C₆)alkyl-SO-(C₁-C₆)alkoxy, tetrahydropyranyloxy, (C₁-C₆)alkylcarbonyl(C₁-C₆)-alkoxy, (C₁-C₆)-alkoxycarbonyl, (C₁-C₆)alkylcarbonyloxy(C₁-C₆)-alkoxy, -SO₂NH₂, phenoxy, 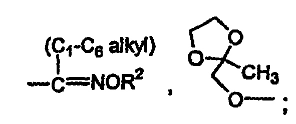 or adjacent R⁵ substituents together are -O-CH₂-O, -O-CH₂CH₂-O-, -O-CF₂-O- or -O-CF₂CF₂-O- and form a ring with the carbon atoms to which they are attached;
R⁶ is (C₁-C₆)alkyl, R⁵-phenyl, R⁵-phenyl(C₁-C₆)alkyl, thienyl, pyridyl, (C₃-C₆)-cycloalkyl, (C₁-C₆)alkyl-OC(O)-NH-(C₁-C₆)alkyl-, di-((C₁-C₆)alkyl)aminomethyl, or 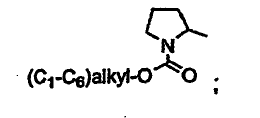 R⁷ is (C₁-C₆)alkyl, R⁵ phenyl or R⁵ phenyl(C₁-C₆)alkyl;
R⁸ is hydrogen or C₁-C₈ alkyl; or R⁷ and R⁸ together are -(CH₂)ₚ-A-(CH₂)_{q}, wherein p and q are independently 2 or 3 and A is a bond, -CH₂-, -S- or -O-, and form a ring with the nitrogen to which they are attached;
R⁹ is 1-2 groups independently selected from hydrogen, C₁-C₆ alkyl, hydroxy, C₁-C₆ alkoxy, halogen, -CF₃ and (C₁-C₆)alkoxy(C₁-C₆)alkoxy;
R¹⁰ is 1 to 5 substituents independently selected from the group consisting of hydrogen, halogen, C₁-C₆ alkyl, hydroxy, C₁-C₆ alkoxy, -CN, -NH₂, C₁-C₆alkylamino, di-((C₁-C₆)alkyl)amino, -CF₃, -OCF₃ and -S(O)₀₋₂(C₁-C₆)alkyl;
R¹¹ is H, C₁-C₆ alkyl, phenyl, benzyl, C₂-C₆ alkenyl, C₁-C₆ alkoxy(C₁-C₆)alkyl, di-((C₁-C₇)alkyl)amino(C₁-C₆)alkyl, pyrrolidinyl(C₁-C₆)alkyl or piperidino(C₁-C₆)alkyl;
R¹² is H or C₁-C₆ alkyl; and
R¹³ is (C₁-C₆)alkyl-C(O)- or (C₁-C₆)alkyl-SO₂-.

2. The use of claim 1 wherein the adenosine A2a receptor antagonist is selected from the group consisting of compounds of the formula 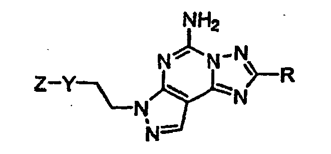 wherein R and Z-Y are as defined in the following table:
| Z-Y- | R |
|---|---|
| 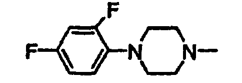 | 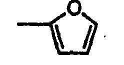 |
| 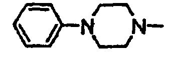 | 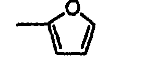 |
| 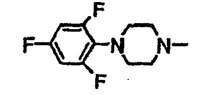 | 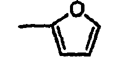 |
| 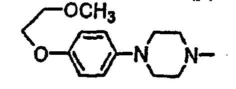 | 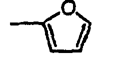 |
| 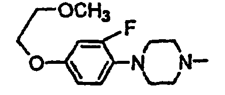 | 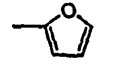 |
| 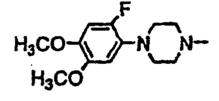 | 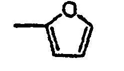 |
| 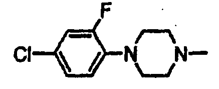 | 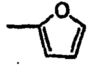 |
| 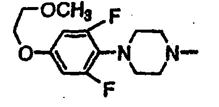 | 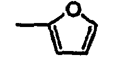 |
| 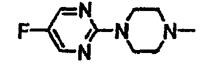 | 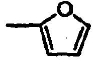 |
| 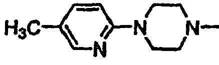 | 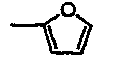 |
| 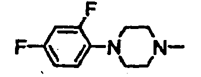 | 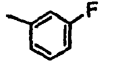 |
| 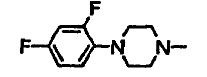 | 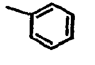 |
or a pharmaceutically acceptable salt or solvate thereof.

3. The use of Claim 2 wherein the adenosine A2a receptor antagonist is 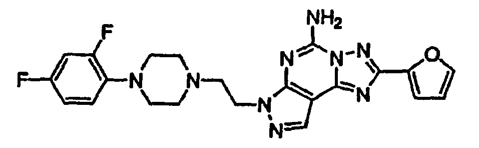 or a pharmaceutically acceptable salt or solvate thereof.

4. The use of claim 1 wherein the Extra-Pyramidal Syndrome has been caused by treatment with a typical antipsychotic agent or an atypical antipsychotic agent

5. The use of claim 4 wherein the typical antipsychotic agent is selected from the group consisting of loxapine, haloperidol, chlorpromazine, prochlorperazine and thiothixene, and the atypical antipsychotic agent is selected from the group consisting of clozapine, olanzapine, loxapine, quetiapine, ziprasidone and risperidone

6. The use of claim 4 further comprising the use of an antipsychotic agent for the preparation of a medicament for use in combination with the adenosine A2a receptor antagonist medicament.

7. The use of claim 6 wherein the antipsychotic agent is a typical antipsychotic agent selected from the group consisting of loxapine, hatoperidol, chlorpromazine, prochlorperazine and thiothixene, or an atypical antipsychotic agent selected from the group consisting of clozapine, olanzapine, loxapine, quetiapine, ziprasidone and risperidone.

8. A kit comprising, in separate containers in a single package, pharmaceutical compositions for use In combination to treat or prevent EPS caused by treatment with antipsychotic agent, wherein one container comprises a pharmaceutical composition comprising an effective amount of an adenosine A₂ₐ receptor antagonist, which is a compound of formula 1 as defined in claim 1, in a pharmaceutically acceptable carrier, and wherein, a separate container comprises a pharmaceutical composition comprising an effective amount of an antipsychotic agent.

9. The use of claim 1 wherein the adenosine A2a receptor antagonist is
selected from the group consisting of compounds of the formula 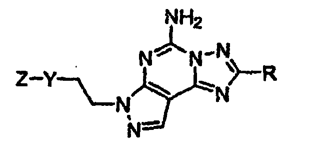 wherein R and Z-Y are as defined In the following table:
| Z-Y- | R |
|---|---|
| 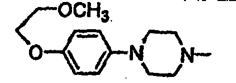 | 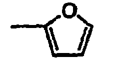 |

## Patentansprüche

1. Die Verwendung eines Adenosin-A2a-Rezeptor-Antagonisten zur Herstellung eines Medikaments zur Behandlung oder Prävention von extrapyramidalem Syndrom (EPS) bei einem Patienten, der mit einem Antipsychotikum behandelt wurde, welches die Nebenwirkung besitzt, EPS hervorzurufen, wobei der Adenosin-A2a-Antagonist eine Verbindung der Formel 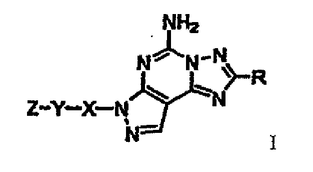 oder ein pharmazeutisch annehmbares Salz davon ist, wobei
R R¹-Furanyl, R¹-Thienyl, R¹-Pyridyl, R¹-Pyridyl-N-oxid, R¹-Oxazolyl, R¹⁰-Phenyl, R¹-Pyrrolyl oder C₄-C₆-Cycloalkenyl ist,
X C₂-C₆-Allcylen oder -C(O)CH₂- ist,
Y -N(R²)CH₂CH₂N(R³)-, -OCH₂CH₂N(R²)-, -O-, -S-, -CH₂S-, -(CH₂)₂-NH- oder 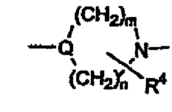 ist
und
Z R⁵-Phenyl, R⁵-Phenyl(C₁-C₆)alkyl, R⁵-Heteroaryl, Diphenylmethyl, R⁶-C(O)-, R⁶-SO₂-,
R⁶-OC(O)-, R⁷-N(R⁸)-C(O)-, R⁷-N(R⁸)-C(S)-, 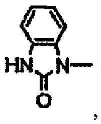 Phenyl-CH(OH)- oder Phenyl-C(=NOR²)- ist, oder, wenn Q 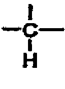 ist, Z auch Phenylamino oder Pyridylamino ist, oder
Z und Y zusammen 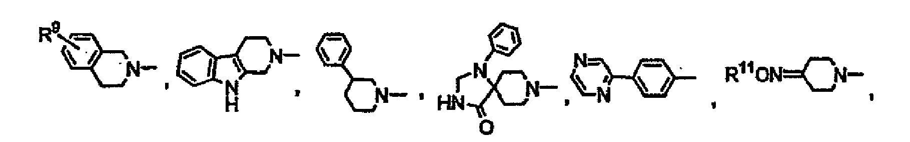 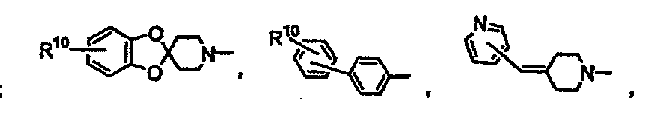 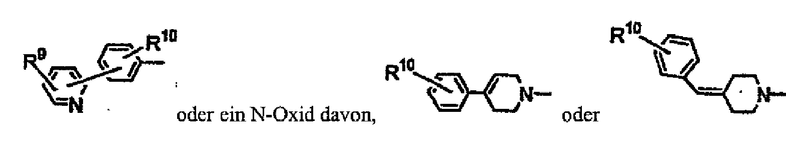 sind,
R¹ 1 bis 3 Substituenten ist, unabhängig ausgewählt aus Wasserstoff, C₁-C₆-Alkyl, -CF₃, Halogen, -NO₂, -NR¹²R¹³, C₁-C₆-Alkoxy, C₁-C₆-Alkylthio, C₁-C₆-Alkylsulfinyl und C₁-C₆-Alkylsulfonyl,
R² und R³ unabhängig ausgewählt sind aus der Gruppe, bestehend aus Wasserstoff und C₁-C₆-Alkyl,
m und n unabhängig 2-3 sind,
Q 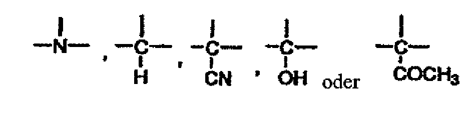 ist,
R⁴ 1-2 Substituenten ist, unabhängig ausgewählt aus der Gruppe, bestehend aus Wasserstoff und C₁-C₆-Alkyl, oder zwei R⁴-Substituenten am selben Kohlenstoff=O bilden können,
R⁵ 1 bis 5 Substituenten ist, unabhängig ausgewählt aus der Gruppe, bestehend aus Wasserstoff, Halogen, C₁-C₆-Alkyl, Hydroxy, C₁-C₆-Alkoxy, -CN, Di-((C₁-C₆)alkyl)amino, -CF₃, -OCF₃, Acetyl, NO₂, Hydroxy(C₁-C₆)alkoxy, (C₁-C₆)Alkoxy(C₁-C₆)alkoxy, Di-((C₁-C₆)alkoxy)(C₁-C₆)alkoxy, (C₁-C₆)Alkoxy(C₁-C₆)alkoxy-(C₁-C₆)alkoxy, Carboxy(C₁-C₆)alkoxy, (C₁-C₆)Alkoxycarbonyl(C₁-C₆)alkoxy, (C₃-C₆)Cycloalkyl(C₁-C₆)alkoxy, Di-((C₁-C₆)alkyl)amino(C₁-C₆)alkoxy, Morpholinyl, (C₁-C₆)Alkyl-SO₂-, (C₁-C₆)Alkyl-SO-(C₁-C₆)alkoxy, Tetrahydropyranoyloxy, (C₁-C₆)Alkylcarbonyl(C₁-C₆)alkoxy, (C₁-C₆)Alkoxycarbonyl, (C₁-C₆)Alkylcarbonyloxy(C₁-C₆)alkoxy, -SO₂NH₂, Phenoxy, 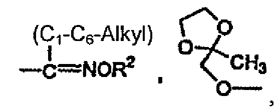 oder benachbarte R⁵-Substituenten zusammen -O-CH₂-O-, -O-CH₂CH₂-O-, -O-CF₃-O- oder -O-CF₂CF₂-O- sind und mit den Kohlenstoffatomen, an die sie gebunden sind, einen Ring bilden,
R⁶ (C₁-C₆)Alkyl, R⁵-Phenyl, R⁵-Phenyl(C₁-C₆)alkyl, Thienyl, Pyridyl, (C₃-C₆)Cycloalkyl, (C₁-C₆)Alkyl-O(C(O)-NH-(C₁-C₆)alkyl-, Di-((C₁-C₆)alkyl)aminomethyl oder 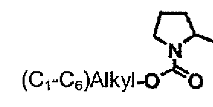 ist,
R⁷ (C₁-C₆)Alkyl, R⁵-Phenyl oder R⁵-Phenyl(C₁-C₆)alkyl ist,
R⁸ Wasserstoff oder C₁-C₈-Alkyl ist, oder R⁷ und R⁸ zusammen -(CH₂)ₚ-A-(CH₂)_{q} sind, wobei p und q unabhängig 2 oder 3 sind und A eine Bindung, -CH₂-, -S- oder -O- ist, und mit dem Stickstoff, an den sie gebunden sind, einen Ring bilden,
R⁹ 1-2 Gruppen ist, unabhängig ausgewählt aus Wasserstoff, C₁-C₆-Alkyl, Hydroxy, C₁-C₆-Alkoxy, Halogen, -CF₃ und (C₁-C₆)Alkoxy(C₁-C₆)alkoxy,
R¹⁰ 1 bis 5 Substituenten ist, unabhängig ausgewählt aus der Gruppe, bestehend aus Wasserstoff, Halogen, C₁-C₆-Alkyl, Hydroxy, C₁-C₆-Alkoxy, -CN, -NH₂, C₁-C₆-Alkylamino, Di-((C₁-C₆)alkyl)amino, -CF₃, -OCF₃ und -S(O)₀₋₂(C₁-C₆)Alkyl,
R¹¹ H, C₁-C₆-Alkyl, Phenyl, Benzyl, C₂-C₆-Alkenyl, C₁-C₆-Alkoxy(C₁-C₆)alkyl, Di-((C₁-C₆)alkyl)amino(C₁-C₆)alkyl, Pyrrolidinyl(C₁-C₆)alkyl oder Piperidino(C₁-C₆)alkyl ist,
R¹² H oder C₁-C₆-Alkyl ist und
R¹³ (C₁-C₆)Alkyl-C(O)- oder (C₁-C₆)Alkyl-SO₂- ist.

2. Die Verwendung gemäß Anspruch 1, wobei der Adenosin-A2a-Rezeptor-Antagonist ausgewählt ist aus der Gruppe, bestehend aus Verbindungen der Formel 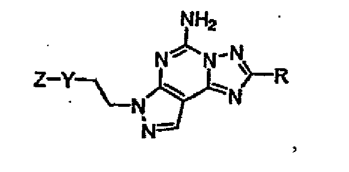 wobei R und Z-Y wie in der folgenden Tabelle definiert sind:
| Z-Y- | R |
|---|---|
| 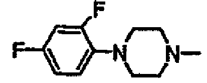 | 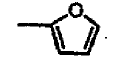 |
| 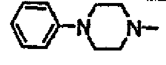 | 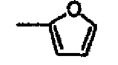 |
| 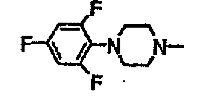 | 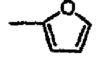 |
| 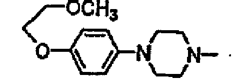 | 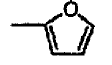 |
| 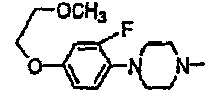 | 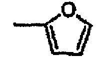 |
| 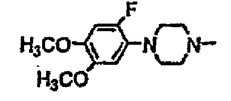 | 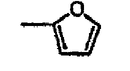 |
| 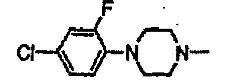 | 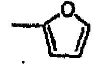 |
| 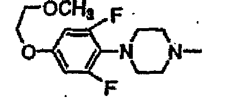 | 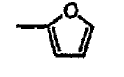 |
| 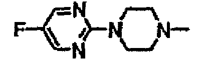 | 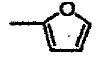 |
| 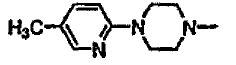 | 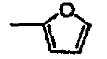 |
| 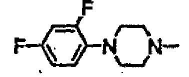 | 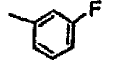 |
| 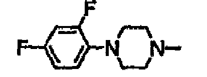 | 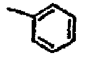 |
oder einem pharmazeutisch annehmbaren Salz oder Solvat davon.

3. Die Verwendung gemäß Anspruch 2, wobei der Adenosin-A2a-Rezeptor-Antagonist 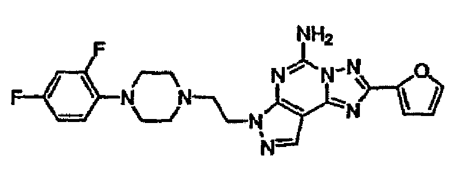 oder ein pharmazeutisch annehmbares Salz oder Solvat davon ist.

4. Die Verwendung gemäß Anspruch 1, wobei das extrapyramidale Syndrom durch Behandlung mit einem typischen Antipsychotikum oder einem atypischen Antipsychotikum hervorgerufen wurde.

5. Die Verwendung gemäß Anspruch 4, wobei das typische Antipsychotikum ausgewählt ist aus der Gruppe, bestehend aus Loxapin, Haloperidol, Chlorpromazin, Prochlorperazin und Thiothixen, und das atypische Antipsychotikum ausgewählt ist aus der Gruppe, bestehend aus Clozapin, Olanzapin, Loxapin, Quetiapin, Ziprasidon und Risperidon.

6. Die Verwendung gemäß Anspruch 4, ferner umfassend die Verwendung eines Antipsychotikums zur Herstellung eines Medikaments zur Verwendung in Kombination mit dem Adenosin-A2a-Rezeptor-Antagonist-Medikament.

7. Die Verwendung gemäß Anspruch 6, wobei das Antipsychotikum ein typisches Antipsychotikum ist, ausgewählt aus der Gruppe, bestehend aus Loxapin, Haloperidol, Chlorpromazin, Prochlorperazin und Thiothixen, oder ein atypisches Antipsychotikum, ausgewählt aus der Gruppe, bestehend aus Clozapin, Olanzapin, Loxapin, Quetiapin, Ziprasidon und Risperidon.

8. Ein Kit, umfassend, in getrennten Behältern in einer Einzelverpackung, pharmazeutische Zusammensetzungen zur Verwendung in Kombination zur Behandlung oder Prävention von EPS, hervorgerufen durch Behandlung mit einem Antipsychotikum, wobei ein Behälter eine pharmazeutische Zusammensetzung enthält, umfassend eine wirksame Menge eines Adenosin-A₂ₐ-Rezeptor-Antagonisten, welcher eine Verbindung der Formel I wie in Anspruch 1 definiert ist, in einem pharmazeutisch annehmbaren Träger, und wobei ein separater Behälter eine pharmazeutische Zusammensetzung enthält, umfassend eine wirksame Menge eines Antipsychotikums.

9. Die Verwendung gemäß Anspruch 1, wobei der Adenosin-A2a-Rezeptor-Antagonist ausgewählt ist aus der Gruppe, bestehend aus Verbindungen der Formel 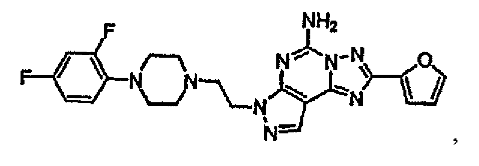 wobei R und Z-Y wie in der folgenden Tabelle definiert sind:
| Z-Y- | R |
|---|---|
| 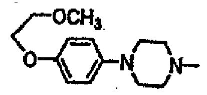 | 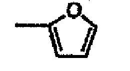 |

## Revendications

1. Utilisation d'un antagoniste récepteur de l'adénosine A2a dans la préparation d'un médicament pour le traitement ou la prévention du syndrome extra-pyramidal (EPS) chez un patient traité avec un agent antipsychotique qui présente l'effet secondaire d'induire l'EPS, où l'antagoniste de l'adénosine A2a est un composé de la formule 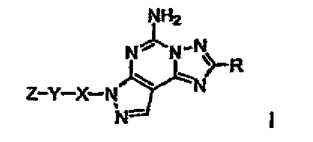 ou un sel pharmaceutiquement acceptable de celui-ci, où
R est R¹-furanyle, R¹-thiényle, R¹-pyridyle, R¹-pyridyle-N-oxyde, R¹-oxazolyle, R¹⁰-phényle, R¹-pyrrolyle ou cycloalcényle C₄-C₆;
X est alkylène C₂-C₆ ou -C(O)CH₂-;
Y est -N(R²)CH₂CH₂N(R³)-, -OCH₂CH₂N(R²)-, -O-, -S-, -CH₂S-, -(CH₂)₂-NH- ou 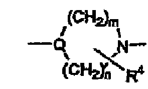 et
Z est R⁵-phényle, R⁵-phénylalkyle (C₁-C₆), R⁵-hétéroaryle, diphénylméthyle, R⁸-C(O)-,
R⁶-SO₂₋, R⁶-OC(O)-, R⁷-N(R⁸)-C(O)-, R⁷-N(R⁸)-C(S)-, 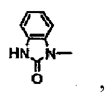 phényle-CH(OH)- ou phényle-C(=NOR²)-; ou bien lorsque Q est 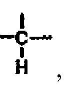 Z est aussi phénylamino ou pyridylamino;
ou
Z et Y ensemble sont 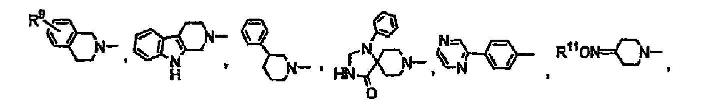 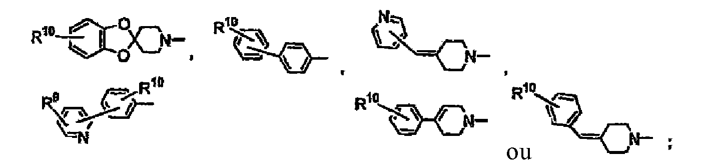 ou un N-oxyde de ceux-ci,
R¹ est 1 à 3 substituants sélectionnés indépendamment parmi hydrogène, alkyle C₁-C₆, -CF₃, halogène, -NO₂, -NR¹²R¹³, alcoxy C₁-C₆, alkylthio C₁-C₆, alkylsulfinyle C₁-C₆ et alkylsulfonyle C₁-C₆;
R² et R³ sont sélectionnés indépendamment parmi le groupe consistant en hydrogène et alkyle C₁-C₆;
m et n sont indépendamment 2-3;
Q est 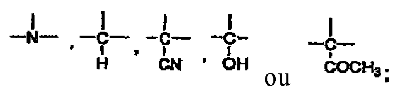 R⁴ est 1-2 substituants sélectionnés indépendamment parmi le groupe consistant en hydrogène et alkyle C₁-C₆, ou bien deux substituants R⁴ sur le même atome de carbone peuvent former =O;
R⁵ est 1 à 5 substituants sélectionnés indépendamment parmi le groupe consistant en hydrogène, halogène, alkyle C₁-C₆, hydroxy, alcoxy C₁-C₆, -CN, di-(alkyle (C₁-C₆))-amino, -CF₃, -OCF₃, acétyle, -NO₂, hydroxyalcoxy (C₁-C₆), alcoxy ( C₁-C₆)-alcoxy (C₁-C₆), di-(alcoxy (C₁-C₆))-alcoxy (C₁-C₆), alcoxy (C₁-C₆)-alcoxy (C₁-C₆)-alcoxy (C₁-C₆), carboxy-alcoxy (C₁-C₆), alcoxy (C₁-C₆)-carbonyle-alcoxy (C₁-C₆), cycloalkyle (C₃-C₆)-alcoxy (C₁-C₆), di(alkyle (C₁-C₆))-amino-alcoxy (C₁-C₆), morpholinyle, alkyle (C₁-C₆)-SO₂-, alkyle (C₁-C₆)-SO-alcoxy (C₁-C₆), tétrahydropyranyloxy, alkyle (C₁-C₆)-carbonyl-alcoxy (C₁-C₆), alcoxy (C₁-C₆)-carbonyle, alkyle (C₁-C₆)-carbonyloxy-alcoxy (C₁-C₆), - SO₂NH₂, phénoxy, 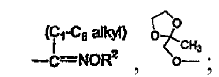 ou bien des substituants R⁵ adjacents sont ensemble -O-CH₂-O-, -O-CH₂CH₂-O-, -O-CF₂-O- ou -O-CR₂CF₂-O- et forment un cycle avec les atomes de carbone auxquels ils sont attachés;
R⁶ est alkyle (C₁-C₆), R⁵-phényle, R⁵-phénylalkyle (C₁-C₆), thiényle, pyridyle, cycloalkyle-(C₃-C₆), alkyle (C₁-C₆)-OC(O)-NH-alkyle (C₁-C₆), di(alkyle(C₁-C₆))-aminométhyle, ou 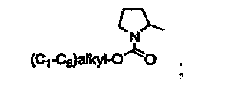 R⁷ est alkyle (C₁-C₆), R⁵-phényle ou R⁵-phénylalkyle (C₁-C₆);
R⁸ est hydrogène ou alkyle C₁-C₆, ou bien R⁷ et R⁸ sont ensemble -(CH₂)ₚ-A-(CH₂)_{q}, où p et q sont indépendamment 2 ou 3 et A est une liaison, -CH₂-, -S- ou -O- et forment un cycle avec l'azote auquel ils sont attachés;
R⁹ est 1-2 groupes sélectionnés indépendamment parmi hydrogène, alkyle C₁-C₆, hydroxy, alcoxy C₁-C₆, halogène, -CF₃ et alcoxy (C₁-C₆)-alcoxy (C₁-C₆);
R¹⁰ est 1 à 5 substituants sélectionnés indépendamment parmi le groupe consistant en hydrogène, halogène, alkyle C₁-C₆, hydroxy, alcoxy C₁-C₆, -CN, -NH₂, alkylamino C₁-C₆, di-(alkyle(C₁-C₆))-amino, -CF₃, -OCF₃ et -S(O)₀₋₂alkyle (C₁-C₆);
R¹¹ est H, alkyle C₁-C₆, phényle, benzyle, alcényle C₂-C₆, alcoxy C₁-C₆-alkyle (C₁-C₆), di-(alkyle (C₁-C₆))-aminoalkyle (C₁-C₆), pyrrolidinyl-alkyle (C₁-C₆) ou pipéridino-alkyle (C₁-C₆);
R¹² est H ou alkyle C₁-C₆; et
R¹³ est alkyle (C₁-C₆)-C(O)- ou alkyle (C₁-C₆)-SO₂-.

2. L'utilisation de la revendication 1, où l'antagoniste récepteur de l'adénosine A2a est sélectionné parmi le groupe consistant en composés de la formule 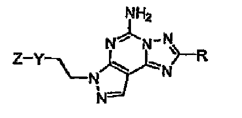 où R et Z-Y sont tels que définis dans le tableau suivant:
| Z-Y- | R |
|---|---|
| 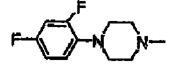 | 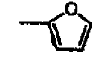 |
| 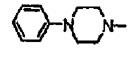 | 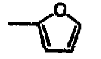 |
| 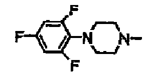 | 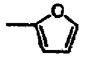 |
| 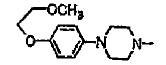 | 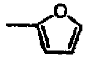 |
| 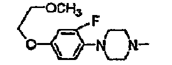 | 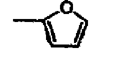 |
| 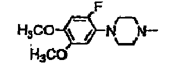 | 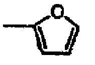 |
| 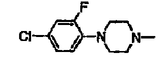 | 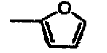 |
| 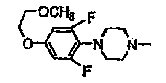 | 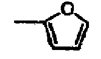 |
| 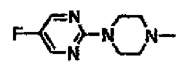 | 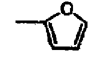 |
| 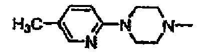 | 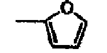 |
| 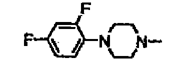 | 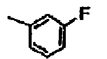 |
| 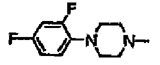 | 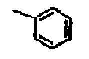 |
ou un sel ou un solvate pharmaceutiquement acceptable de ceux-ci.

3. L'utilisation de la revendication 2, où l'antagoniste récepteur de l'adénosine A2a est 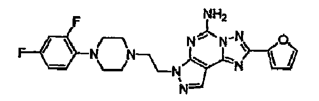 ou un sel ou un solvate pharmaceutiquement acceptable de celui-ci.

4. L'utilisation de la revendication 1, où le syndrome extra-pyramidal a été causé par un traitement avec un agent antipsychotique typique ou un agent antipsychotique atypique.

5. L'utilisation de la revendication 4, où l'agent antipsychotique typique est sélectionné parmi le groupe consistant en loxapine, halopéridol, chlorpromazine, prochlorperazine et thiothixène, et l'agent antipsychotique atypique est sélectionné parmi le groupe consistant en clozapine, olanzapine, loxapine, quétiapine, ziprasidone et rispéridone.

6. L'utilisation de la revendication 4, comprenant en outre l'utilisation d'un agent antipsychotique dans la préparation d'un médicament à utiliser en combinaison avec le médicament antagoniste récepteur de l'adénosine A2a.

7. L'utilisation de la revendication 6, où l'agent antipsychotique est un agent antipsychotique typique sélectionné parmi le groupe consistant en loxapine, halopéridol, chlorpromazine, prochlorperazine et thiothixène, ou un agent antipsychotique atypique sélectionné parmi le groupe consistant en clozapine, olanzapine, loxapine, quétiapine, ziprasidone et rispéridone.

8. Kit comprenant, dans des récipients séparés dans un seul emballage, des compositions pharmaceutiques à utiliser en combinaison dans le traitement ou la prévention d'EPS causé par un traitement avec un agent antipsychotique, dans lequel un récipient comprend une composition pharmaceutique comprenant une quantité efficace d'un antagoniste récepteur de l'adénosine A2a qui est un composé de la formule 1 tel que défini dans la revendication 1, dans un excipient pharmaceutiquement acceptable, et dans lequel un récipient séparé comprend une composition pharmaceutique comprenant une quantité efficace d'un agent antipsychotique.

9. L'utilisation de la revendication 1, où l'antagoniste récepteur de l'adénosine A2a est sélectionné parmi le groupe consistant en composés de la formule 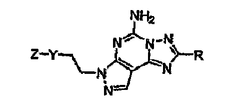 où R et Z-Y sont tels que définis dans le tableau suivant:
| Z-Y- | R |
|---|---|
| 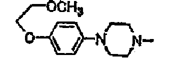 | 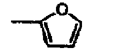 |
